# EUROPEAN PATENT APPLICATION

(11) **EP 4 818 163 A1**
(43) Date of publication of application: **07.10.2026**
(21) Application number: 24896580.8
(22) Date of filing: 27.11.2024
(51) Int. Cl.: C07D 277/20, C07D 295/02, C07D 213/02, C07D 213/04, A61K 45/06, A61P 31/14, A61K 31/496

(54) **POLYCYCLIC COMPOUND AND USE THEREOF**

(30) Priority: 27.11.2023 CN 202311591961
(71) Applicant: Zhejiang Jingke Pharmaceuticals Co., Ltd., Huzhou, Zhejiang 313100 (CN)
(72) Inventor: LI, Xinjian, Changxing County Huzhou, Zhejiang 313100 (CN); LIANG, Bin, Changxing County Huzhou, Zhejiang 313100 (CN)
(74) Representative: Engelhard, Markus
(86) International application number: PCT/CN2024/134944
(87) International publication number: WO 2025/113494

(57) **Abstract**

The present disclosure belongs to the technical field of medicine, and particularly discloses a polycyclic compound as represented by formula I and use thereof. The polycyclic compound of the present disclosure has a structure as represented by formula I, and definitions of each group and substituent are as described in the specification. The compound of the present disclosure can be used for treating or preventing neurodegenerative diseases such as Alzheimer's disease, Parkinson's disease, progressive multiple sclerosis, and amyotrophic lateral sclerosis.

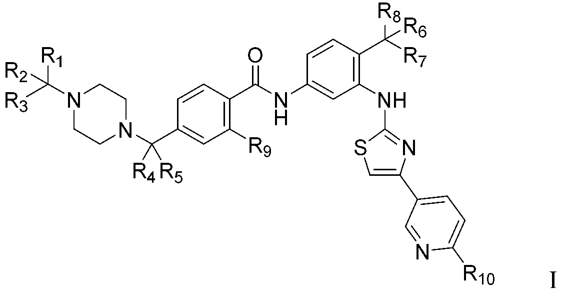

## Description

### TECHNICAL FIELD

The present disclosure relates to the technical field of medicine, particularly discloses a polycyclic compound as represented by formula I, and further discloses use thereof.

### BACKGROUD

Alzheimer's disease, commonly known as senile dementia, is a common neurodegenerative disease, with symptoms of memory decline, neuronal death, and the like, and a main clinical marker of neurofibrillary tangles. A pathological change process of Alzheimer's disease is considerably complex. At present, effective therapeutic drugs for Alzheimer's disease are being researched worldwide, yet with limited achievement. There are currently no specific therapeutic drugs capable of treating or reversing disease progression of Alzheimer's disease. More than a decade ago, the U.S. FDA approved a total of five therapeutic compounds falling into 2 categories, including cholinesterase inhibitors and NMDA receptor antagonists. However, the therapeutic compounds can only temporarily alleviate the disease symptoms and cannot halt the disease progression.

It is currently widely accepted that the primary pathogenesis of Alzheimer's disease involves β-amyloid protein deposition in brain tissue, cerebrovascular structural abnormalities leading to activation of microglia and astrocytes in brain tissue, which further causes inflammatory mediators, increased generation of free radicals in brain tissue accompanied by reduced antioxidant capacity, an imbalance between an acetylcholine system and an anti-acetylcholine system in brain tissue, and the like, all of which lead to structural and functional damage to brain tissue in patients with senile dementia, increased levels of inflammatory mediators in hippocampus, and reduced antioxidant capacity in hippocampus, and ultimately trigger Alzheimer's disease. Several pharmaceutical companies have developed various vaccines targeting Abeta protein and phosphorylation inhibitors for multiple enzymes involved in formation of the protein. Nevertheless, all these attempts have so far ended in failure due to lack of efficacy. It is believed that reducing the toxicity of the Abeta protein after the disease onset in the patients is already too late, so that therapeutic drugs should be developed by reducing an amount of the protein.

### SUMMARY

The present disclosure is intended to develop a polycyclic compound as represented by formula I, and to provide use thereof in the manufacture of a medicament at the same time, and the compound provided by the present disclosure may be used in the manufacture of a medicament for treating diseases such as neurodegenerative disease.

Specifically, in a first aspect, the present disclosure provides a polycyclic compound, wherein the compound is a compound as represented by formula I, or a pharmaceutically acceptable salt, stereoisomer, tautomer, hydrate, solvate, isotopic compound or prodrug thereof: 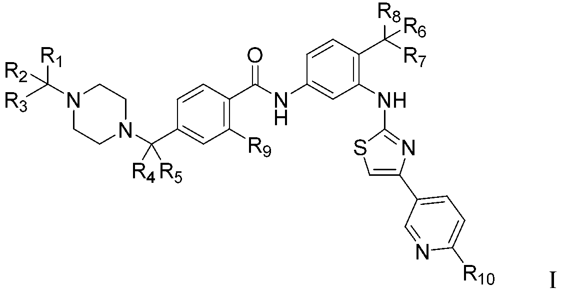 wherein:
R₁, R₂, R₃, R₄ and R₅ each independently represent H or D, and at least one of R₁, R₂, R₃, R₄ and R₅ is D;
R₆, R₇ and R₈ each independently represent H, D or F; and
R₉ and R₁₀ each independently represent H or F.

In one embodiment of the present disclosure, in formula I, at least two of R₁, R₂, R₃, R₄, R₅, R₆, R₇ and R₈ represent D.

In one embodiment of the present disclosure, in formula I, R₁, R₂ and R₃ are all H, or are all D.

In one embodiment of the present disclosure, in formula I, R₄ and R₅ are both H, or are both D.

In one embodiment of the present disclosure, in formula I, R₆, R₇ and R₈ are all H, or are all D, or are all F.

In one embodiment of the present disclosure, in formula I, R₉ and R₁₀ are both H, or either one of them is F.

In one embodiment of the present disclosure, the compound of the present disclosure is selected from the group consisting of the compounds represented by any one of the following structural formulas:

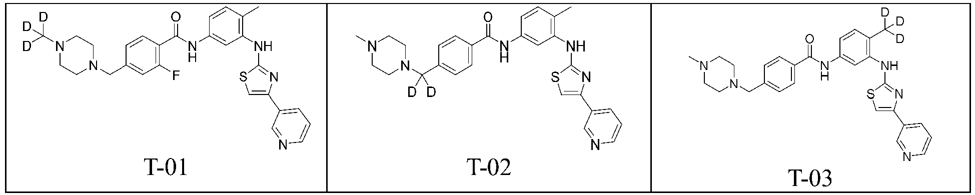

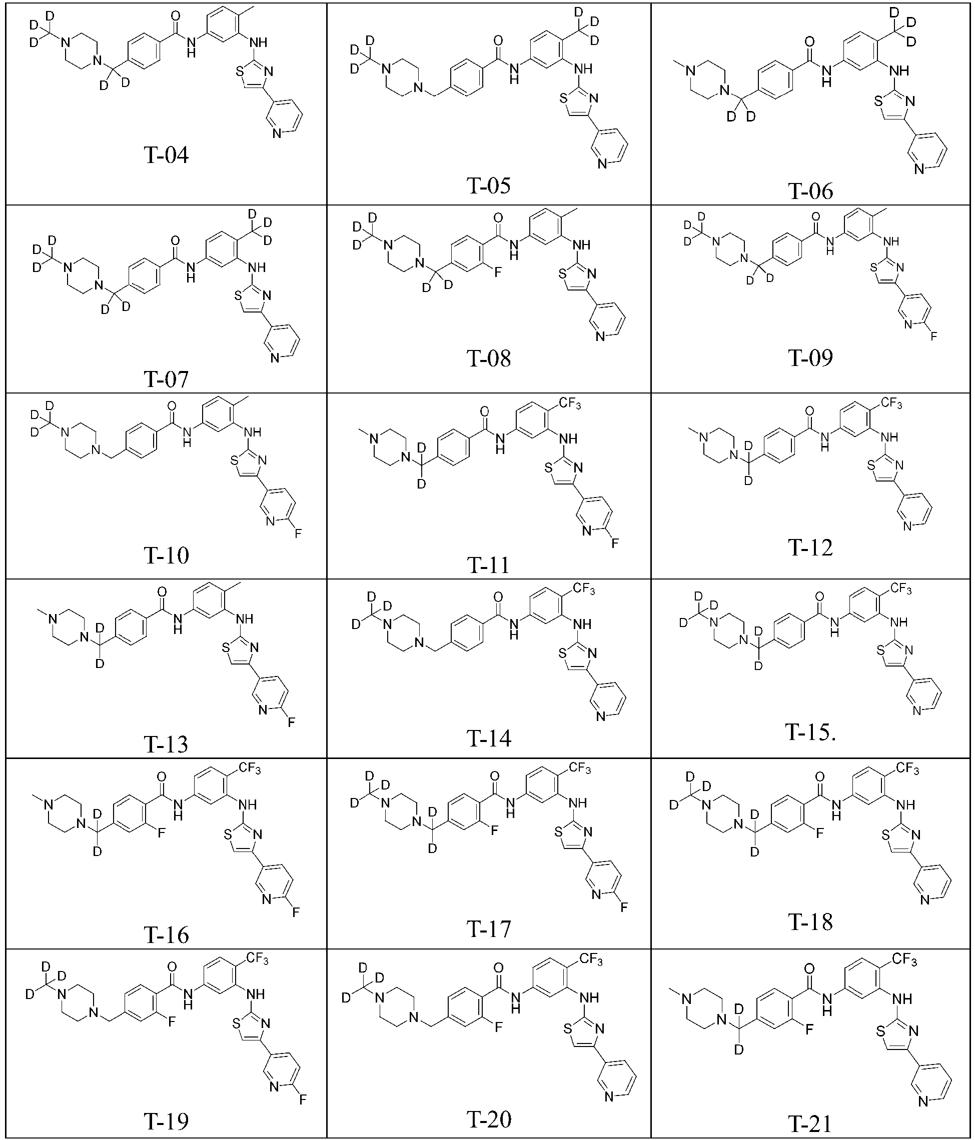

In a second aspect, the present disclosure provides a pharmaceutical composition, wherein the pharmaceutical composition comprises a prophylactically and/or therapeutically effective amount of the compound of the present disclosure, and a pharmaceutically acceptable carrier.

In a third aspect, the present disclosure provides use of the compound of the present disclosure or the pharmaceutical composition of the present disclosure.

The compound of the present disclosure or the pharmaceutical composition of the present disclosure is used in the manufacture of a medicament for treating a disease selected from the group consisting of neurodegenerative disease, cancer, progressive supranuclear palsy, mast cell activation syndrome (MCAS), rheumatoid arthritis, asthma, Crohn's disease, sickle cell disease, and coronavirus disease 2019 (COVID-19).

In one embodiment of the present disclosure, the neurodegenerative disease in the present disclosure is selected from the group consisting of Alzheimer's disease, Parkinson's disease, progressive multiple sclerosis, and amyotrophic lateral sclerosis.

In one embodiment of the present disclosure, the cancer in the present disclosure is selected from the group consisting of prostate cancer, pancreatic cancer, gastric cancer, liver cancer, multiple myeloma, and colorectal cancer.

The novel compound provided by the present disclosure may be used in the manufacture of a medicament for treating diseases such as neurodegenerative disease, and particularly has a prominent use in a medicament for the treatment or prevention of Alzheimer's disease, Parkinson's disease, progressive multiple sclerosis, amyotrophic lateral sclerosis, and the like, and exhibits superior pharmacodynamic and pharmacokinetic properties, and the compound of the present disclosure has lower toxic and side effects.

It should be understood that, within the scope of the present disclosure, the above technical features of the present disclosure and the technical features described in detail hereinafter (such as the examples) may be combined with each other to form new or preferred technical solutions. Due to space limitations, the technical solutions are not repeatedly described herein one by one.

### DESCRIPTION OF EXEMPLARY EMBODIMENTS

After extensive and in-depth research, the present inventors have unexpectedly discovered a class of compounds with excellent therapeutic effects on cancers and neurodegenerative diseases. In addition, the compounds have favorable pharmacodynamic/pharmacokinetic properties. On this basis, the present disclosure has been accomplished.

### Terms

Unless otherwise specified, the following terms used in the present disclosure (including the specification and the claims) have the definitions set forth below.

When a substituent is described by a conventional chemical formula written from left to right, the substituent also includes a chemically equivalent substituent obtained when the structural formula is written from right to left. For example, -CH₂O- is equivalent to -OCH₂-.

"Alkyl (used alone or as a part of another group)" refers to a monovalent linear or branched saturated hydrocarbon group consisting solely of carbon and hydrogen atoms and containing 1 to 12 carbon atoms. The alkyl is preferably C1-C6 alkyl (which refers to an alkyl containing 1, 2, 3, 4, 5 or 6 carbon atoms). Examples of the alkyl group comprise, but are not limited to, methyl, ethyl, propyl, isopropyl, isobutyl, sec-butyl, tert-butyl, pentyl, n-hexyl, octyl, dodecyl, and the like. In the present disclosure, the alkyl is also intended to comprise substituted alkyl, which refers to an alkyl in which one or more positions are substituted, particularly with 1-4 substituents at any position. "Haloalkyl" refers to the alkyl as defined herein, in which one or more hydrogen atoms are substituted with identical or different halogen atoms. Examples of the haloalkyl comprise -CH₂Cl, -CH₂CF₃, -CH₂CCl₃, perfluoroalkyl (such as -CF₃), and the like.

"Alkylene" refers to a divalent group of alkyl, such as CH₂-, -CH₂CH₂- and -CH₂CH₂CH₂-.

"Alkoxy (used alone or as a part of another group)" refers to alkyl linked to an oxy group, which has a structure alkyl-O- , wherein the alkyl is as defined above, and preferably, the alkoxy is C1-C6 alkoxy. The alkoxy comprises, but is not limited to, methoxy, ethoxy, propoxy, tert-butoxy, and the like. "Haloalkoxy" refers to a group of formula -OR, wherein R is the haloalkyl group as defined herein. Examples of the haloalkoxy group comprise, but are not limited to, trifluoromethoxy, difluoromethoxy, 2,2,2-trifluoroethoxy, and the like.

"Thioalkyl" refers to alkyl in which carbon is substituted with S, S(O) or S(O)₂.

"Alkenyl (used alone or as a part of another group)" refers to an aliphatic group containing at least one double bond, which generally has 2 to 20 carbon atoms. In the present disclosure, "C2-C6 alkenyl" refers to alkenyl containing 2, 3, 4, 5 or 6 carbon atoms. The alkenyl comrpises, but is not limited to, vinyl, propenyl, butenyl, 1-methylbut-2-en-1-yl, and the like. In the present disclosure, the alkenyl comprises substituted alkenyl.

"Alkenylene" refers to alkenyl having two attachment points. For example, "ethenylene" represents a group -CH=CH-. The alkenylene may also be unsubstituted or substituted with one or more substituents.

"Alkynyl (used alone or as a part of another group)" refers to a linear or branched hydrocarbon chain containing more than 2 carbon atoms and characterized by having one or more triple bonds, which generally has 2 to 20 carbon atoms. In the present disclosure, "C2-C6 alkynyl" refers to alkynyl having 2, 3, 4, 5 or 6 carbon atoms. The alkynyl comprises, but is not limited to, ethynyl, propargyl and 3-hexynyl. One of the triple-bonded carbon atoms may optionally serve as an attachment point of an alkynyl substituent. In the present disclosure, the alkynyl further comprises substituted alkynyl.

"Alkynylene" refers to alkynyl having two attachment points. For example, "ethynylene" represents a group: -C≡C-. The alkynylene may also be in unsubstituted or substituted with one or more substituents.

"Aliphatic group" refers to linear, branched or cyclic hydrocarbyl, comprising saturated and unsaturated groups, such as alkyl, alkenyl and alkynyl.

"Aromatic ring system" refers to a monocyclic, bicyclic or polycyclic hydrocarbon ring system, wherein at least one ring is aromatic.

"Aryl (used alone or as a part of another group)" refers to a monovalent group of an aromatic ring system. Representative aryl comprises a fully aromatic ring system, such as phenyl, naphthyl and anthracenyl; and a ring system in which an aromatic carbocyclic ring is fused with one or more non-aromatic carbocyclic rings, such as indanyl, phthalimidyl, naphthalimidyl, tetrahydronaphthyl, or the like. In the present disclosure, the aryl is preferably C6-C12 aryl. In the present disclosure, the aryl is also intended to comprise substituted aryl.

"Arylalkyl" or "aralkyl" refers to an alkyl moiety in which a hydrogen atom of the alkyl is substituted with aryl. The aralkyl comprises a group in which one or more hydrogen atoms are substituted with aryl, and the aryl and the alkyl are as defined above. Examples of "arylalkyl" or "aralkyl" comprise benzyl, 2-phenylethyl, 3-phenylpropyl, 9-fluorenyl, diphenylmethyl, trityl, and the like.

"Aryloxy" refers to -O-(aryl), wherein the aryl moiety is as defined herein.

"Heteroalkyl" refers to substituted alkyl having one or more skeletal chain atoms selected from atoms other than carbon, such as oxygen, nitrogen, sulfur, phosphorus, or combinations thereof. A numerical range may be specified, for example, C1-C6 heteroalkyl refers to a number of carbon atoms in a chain, which includes 1 to 6 carbon atoms. For example, a group -CH₂OCH₂CH₃ is designated as "C3" heteroalkyl. Attachment to the remainder of the molecule may be realized through a heteroatom or a carbon atom in a heteroalkyl chain. "Heteroalkylene" refers to optionally substituted divalent alkyl, which contains one or more skeletal chain atoms selected from atoms other than carbon, such as oxygen, nitrogen, sulfur, phosphorus, or combinations thereof.

"Carbocyclic ring system" refers to a monocyclic, bicyclic or polycyclic hydrocarbon ring system, wherein each ring is fully saturated or contains one or more unsaturated units, and none of the rings are aromatic.

"Carbocyclyl" refers to a monovalent group of the carbocyclic ring system, such as comprising cycloalkyl (cyclopentyl, cyclobutyl, cyclopropyl, cyclohexyl, and the like) and cycloalkenyl (cyclopentenyl, cyclohexenyl, cyclopentadienyl, and the like).

"Cycloalkyl" refers to a monovalent saturated carbocyclic group consisting of monocyclic or bicyclic rings, which has 3-12 ring atoms, preferably 3-10 ring atoms, and more preferably 3-8 ring atoms. The cycloalkyl may be optionally substituted with one or more substituents, wherein each substituent is independently hydroxy, alkyl, alkoxy, halogen, haloalkyl, amino, monoalkylamino or dialkylamino. Examples of the cycloalkyl group comprise, but are not limited to, cyclopropyl, cyclobutyl, cyclopentyl, cyclohexyl, cycloheptyl, and the like.

"Cycloalkoxy" refers to a group of formula -OR, wherein R is the cycloalkyl as defined herein. Exemplary cycloalkoxy comprises cyclopropyloxy, cyclobutyloxy, cyclopentyloxy, cyclohexyloxy, and the like. "Cycloalkylalkyl" refers to -(cycloalkyl)-alkyl, wherein the cycloalkyl and the alkyl are as disclosed herein. The "cycloalkylalkyl" is linked to a parent molecular structure via the cycloalkyl.

"Heteroaromatic ring system" refers to a monocyclic (such as 5- or 6-membered), bicyclic (6-12-membered) or polycyclic ring system, in which at least one ring is aromatic and contains at least one heteroatom (such as N, O or S); and none of the other rings are heterocyclic groups (as defined below). In some instances, an aromatic ring containing heteroatoms contains 1, 2, 3 or 4 ring heteroatoms in the ring. At least one ring thereof is heteroaromatic, and the remaining rings may be saturated, partially unsaturated or fully unsaturated.

"Heteroaryl" refers to a monocyclic (such as 5- or 6-membered), bicyclic (such as 8-10-membered) or tricyclic group containing 5 to 12 ring atoms, which comprises at least one aromatic ring having 1, 2 or 3 ring heteroatoms selected from N, O or S, with the remaining ring atoms being carbon. It should be understood that an attachment point of the heteroaryl should be located on the aromatic ring. Examples of the heteroaryl group comprise, but are not limited to: imidazolyl, oxazolyl, isoxazolyl, thiazolyl, isothiazolyl, oxadiazolyl, thiadiazolyl, pyrazinyl, thienyl, furanyl, pyranyl, pyridyl, pyrrolyl, pyrazolyl, pyrimidinyl, quinolinyl, isoquinolinyl, benzofuranyl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthyridinyl, pteridinyl, carbazolyl, azepinyl, diazepinyl, acridinyl, and the like. Heteroarylene refers to heteroaryl having two attachment points.

"Heterocyclic ring system" refers to a monocyclic, bicyclic or polycyclic ring system, in which at least one ring is saturated or partially unsaturated (but non-aromatic), and the ring contains at least one heteroatom. The heterocyclic ring system may be linked to a side group via any heteroatom or carbon atom, which forms a stable structure, and any ring atom may be optionally substituted.

"Heterocyclyl" refers to a monovalent group of the heterocyclic ring system, which generally refers to a stable monocyclic ring (such as 3-8-membered, such as 3-, 4-, 5-, 6-, 7- or 8-membered) or bicyclic ring (such as 5-12-membered, such as 5-, 6-, 7-, 8-, 9-, 10-, 11- or 12-membered) or polycyclic ring (such as 7-14-membered, such as 7-, 8-, 9-, 10-, 11-, 12-, 13- or 14-membered), comprising fused, spiro and/or bridged ring structures, is saturated or partially unsaturated, and contains carbon atoms and 1, 2, 3 or 4 heteroatoms independently selected from N, O, and S. Representative heterocyclyl comprises the following ring systems: (1) each ring is non-aromatic and at least one ring contains heteroatoms, for example, tetrahydrofuryl, tetrahydropyranyl, tetrahydrothienyl, pyrrolidinyl, pyrrolidonyl, piperidinyl, pyrrolinyl, decahydroquinolinyl, oxazolidinyl, piperazinyl, dioxanyl, dioxolanyl, diazepinyl, oxazepinyl, thiazepinyl, morpholinyl and quinuclidinyl; (2) at least one ring is non-aromatic and contains heteroatoms, and at least one other ring is an aromatic carbocyclic ring, for example, 1,2,3,4-tetrahydroquinolinyl and 1,2,3,4-tetrahydroisoquinolinyl; and (3) at least one ring is non-aromatic and contains heteroatoms, and at least one other ring is aromatic and contains heteroatoms, for example, 3,4-dihydro-1H-pyrano[4,3-c]pyridine and 1,2,3,4-tetrahydro-2,6-diazanaphthalene. Heterocyclylene refers to heterocyclyl having two attachment points. In the present disclosure, preferred heterocyclylene is bicyclic, wherein one ring is heteroaryl, and the group is linked to other moieties in the general formula via the heteroaryl. In the present disclosure, preferred heterocyclylene is 5-6-membered monocyclic heterocyclylene or 8-10-membered bicyclic heterocyclylene.

"Heterocyclylalkyl" refers to alkyl substituted with heterocyclyl, wherein the heterocyclyl and the alkyl are as defined above.

"Alkylamino" refers to a group having a structure alkyl-NR-, wherein R is H, or the alkyl, cycloalkyl, aryl or heteroaryl as described above.

"Cycloalkylamino" refers to a group of formula -NRₐR_{b}, wherein Rₐ is H, the alkyl is as defined herein, or the cycloalkyl is as defined herein, and R_{b} is the cycloalkyl as defined herein; or Rₐ and R_{b}, together with N atoms to which they are attached, form 3-10-membered N-containing monocyclic or bicyclic heterocyclyl, such as tetrahydropyrrolyl. As used herein, C3-C8 cycloalkylamino refers to amino containing 3-8 carbon atoms.

In the present disclosure, "ester group" refers to a group having a structure -C(O)-O-R or R-C(O)-O-, wherein R independently represents the hydrogen, alkyl, cycloalkyl, aryl, heteroaryl or heterocyclyl as defined above.

In the present disclosure, the term "amido" refers to a group having a structure -CONRR', wherein R and R' may independently represent the hydrogen, alkyl or substituted alkyl, cycloalkyl or substituted cycloalkyl, aryl or substituted aryl, heterocyclic ring or substituted heterocyclic ring as defined above. R and R' may be the same or different in a dialkylamino moiety.

In the present disclosure, the term "sulfonamido" refers to a group having a structure -SO₂NRR', wherein R and R' may independently represent the hydrogen, alkyl or substituted alkyl, cycloalkyl or substituted cycloalkyl, aryl or substituted aryl, heterocyclic ring or substituted heterocyclic ring as defined above. R and R' may be the same or different in a dialkylamino moiety.

"Ketone carbonyl" refers to R-C(=O)-, wherein R is the alkyl, cycloalkyl, and the like as defined above.

When a substituent is a non-terminal substituent, it is a corresponding divalent subunit of the group. For example, alkyl corresponds to alkylene, cycloalkyl corresponds to cycloalkylene, heterocyclyl corresponds to heterocyclylene, and alkoxy corresponds to alkoxylene.

In the present disclosure, each of the above alkyl, alkoxy, cycloalkyl, heteroalkyl, aryl, heteroaryl, cycloheteroalkyl, alkenyl, alkyne, heterocyclic ring, heterocyclyl, and the like, may be substituted or unsubstituted.

In the present disclosure, the term "substituted" means that one or more hydrogen atoms on a specified group are substituted with a specified substituent. The specified substituent is the substituent described accordingly in the preceding text, or the substituent appearing in each example. Unless otherwise specified, a substituted group may have a substituent selected from a specific group at any substitutable position of the group, and the substituent may be the same or different at each position. Those skilled in the art shall understand that contemplated combinations of substituents in the present disclosure are those stable or chemically feasible combinations. Typical substituents comprise, but are not limited to, one or more of the following groups: for example, hydrogen, deuterium, halogen (such as a monohalogen substituent or a polyhalogen substituent, and the latter is, for example, trifluoromethyl or alkyl containing Cl3), cyano, nitro, oxo (such as =O), trifluoromethyl, trifluoromethoxy, cycloalkyl, alkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein Rₐ may independently represent hydrogen, deuterium, alkyl, cycloalkyl, alkenyl, alkynyl, heterocyclic ring, or aromatic ring; R_{b}, R_{c} and R_{d} may independently represent hydrogen, deuterium, alkyl, cycloalkyl, heterocyclic ring, or aromatic ring, or R_{b} and R_{c}, together with N atoms, may form a heterocyclic ring; and Rₑ may independently represent hydrogen, alkyl, cycloalkyl, alkenyl, alkynyl, heterocyclic ring, or aromatic ring. The aforementioned typical substituent, such as alkyl, cycloalkyl, alkenyl, cycloalkenyl, alkynyl, heterocyclic ring or aromatic ring, may be optionally substituted. The substituents, for example, comprise (but are not limited to): halogen, hydroxy, cyano, carboxyl (-COOH), C1-C6 alkyl, C2-C6 alkenyl, C2-C6 alkynyl, C3-C8 cycloalkyl, 3-12-membered heterocyclyl, aryl, heteroaryl, C1-C8 aldehyde group, C2-C10 acyl, C2-C10 ester group, amino, C1-C6 alkoxy, C1-C10 sulfonyl, C1-C6 ureido, and the like.

"Cyano" refers to a -CN group.

"Nitro" refers to -NO₂.

"Hydroxy" refers to -OH.

"Amino" refers to -NH₂ or RNH-, wherein R is ketone carbonyl, sulfonyl, sulfonamido, Ra-C(=O)-, RaRbN-C(=O)-, and the like, and Ra and Rb are alkyl, cycloalkyl, aryl, heteroaryl, or the like.

"Halogen (halo)" refers to any halogen group, for example, -F, -Cl, -Br or -I.

"Deuteride" refers to a compound obtained by substituting one or more hydrogen atoms (H) with deuterium atoms (D) in the compound.

In the present disclosure, the term "multiple" independently refers to 2, 3, 4 or 5.

A structural formula of carbamate group is -NH-C(=O)-O-R, wherein R is alkyl, aryl, heteroaryl, and the like.

### Active ingredient

As used herein, the term "the compound of the present disclosure" or "the active ingredient of the present disclosure" may be used interchangeably, and refers to a compound as represented by formula I, or a pharmaceutically acceptable salt, hydrate, solvate, isotopic compound (such as deuterated compound) or prodrug thereof. The term further comprises a racemate or an optical isomer.

The compound as represented by formula I has the following structure: 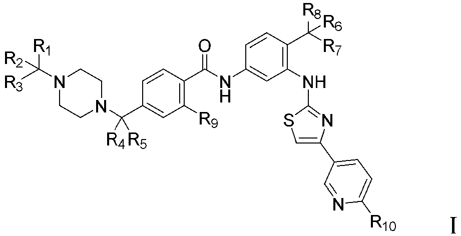 wherein, each group is as defined above.

A salt that the compound of the present disclosure may form is also within the scope of the present disclosure. Unless otherwise specified, the compound of the present disclosure is understood to include the salt thereof. The term "salt" as used herein refers to an acidic or basic salt formed with inorganic or organic acid and base. In addition, when the compound of the present disclosure contains a basic moiety, including, but being not limited to, pyridine or imidazole, and contains an acidic moiety, including, but being not limited to, carboxylic acid, any possible zwitter ion ("internal salt") is included within the scope of the term "salt". A pharmaceutically acceptable salt (non-toxic and physiologically tolerable) is preferred, though other salts may also be useful, for example, in an isolation or purification step during preparation. The compound of the present disclosure may form the salt. For example, a compound I may react with a certain amount, such as an equivalent amount, of acid or a base, and a salt is precipitated in a medium or lyophilized in an aqueous solution.

The basic moiety contained in the compound of the present disclosure, comprises, but is not limited to amine, or pyridine or imidazole ring, and may form a salt with inorganic or organic acid. Typical acids that may achieve salt formation comprise acetate (derived from, for example, acetic acid or trihaloacetic acid, such as trifluoroacetic acid), adipate, alginate, ascorbate, aspartate, benzoate, benzenesulfonate, bisulfate, borate, butyrate, citrate, camphorate, camphorsulfonate, cyclopentanepropionate, digluconate, dodecyl sulfate, ethanesulfonate, fumarate, glucoheptonate, glycerophosphate, hemisulfate, heptanoate, hexanoate, hydrochloride, hydrobromide, hydroiodide, isethionate (for example, 2-hydroxyethanesulfonate), lactate, maleate, methanesulfonate, naphthalenesulfonate (for example, 2-naphthalenesulfonate), nicotinate, nitrate, oxalate, pectinate, persulfate, phenylpropionate (for example, 3-phenylpropionate), phosphate, picrate, pivalate, propionate, salicylate, succinate, sulfate (derived from, for example, sulfuric acid), sulfonate, tartrate, thiocyanate, toluenesulfonate such as p-toluenesulfonate, dodecanoate, and the like.

Acidic moieties that may be contained in some compounds of the present disclosure include, but are not limited to, carboxylic acid, and may form salts with various organic and inorganic bases. Salts formed with typical bases include ammonium salts, alkali metal salts such as sodium, lithium and potassium salts, alkaline earth metal salts such as calcium and magnesium salts, and salts formed with organic bases (for example, organic amine), such as benzathine, dicyclohexylamine, hebamine (salt formed with N,N-di(dehydroabietyl)ethylenediamine), N-methyl-D-glucamine, N-methyl-D-glucanamide, tert-butylamine, and salts formed with amino acids such as arginine, lysine, and the like. A nitrogen-containing basic group may form a quaternary ammonium salt with a halide, such as a small-molecule alkyl halide (for example, chlorides, bromides and iodides of methyl, ethyl, propyl and butyl), dialkyl sulfate (for example, dimethyl sulfate, diethyl sulfate, dibutyl sulfate and dipentyl sulfate), a long-chain alkyl halide (for example, chlorides, bromides and iodides of decyl, dodecyl and tetradecyl), an aralkyl halide (for example, a benzyl chloride and a phenethyl bromide), and the like.

A prodrug and a solvate (or solvated substance) of the compound of the present disclosure are also included within the scope of the present disclosure.

The term "prodrug" herein refers to a compound, which produces the compound of the present disclosure, the salt or the solvate by chemical transformation via a metabolic or chemical process upon the treatment of relevant diseases. The compound of the present disclosure comprises the solvate, such as a hydrate.

The compound of the present disclosure, the salt or the solvate may exist in a tautomeric form (for example, amide and imino ether). All tautomers are a part of the present disclosure.

All stereoisomers of compounds (for example, those arising from asymmetric carbon atoms that may be present due to various substituents), comprising enantiomeric and diastereomeric forms thereof, fall within the contemplated scope of the present disclosure. The individual stereoisomer of the compound of the present disclosure may exist in isolation from other isomers (such as serving as a pure or substantially pure optical isomer with specific activity), or may be present in the form of mixture, such as a racemate, or may form a mixture with all or part of other stereoisomers. A chiral center of the compound of the present disclosure adopts either R or S configuration, as defined by the 1974 recommendations of the International Union of Pure and Applied Chemistry (IUPAC). The racemic form may be resolved by physical means, for example, fractional crystallization, or separation and crystallization via derivatization into a diastereomer, or separation by chiral column chromatography. The individual optical isomer may be obtained from the racemate by suitable methods, comprising, but being not limited to conventional methods, such as recrystallization after salt formation with optically active acid.

As for the compound of the present disclosure, the compound obtained sequentially by preparation, separation and purification has a weight content of 90% or higher, for example, 95% or higher, or 99% or higher ("substantially pure" compound), which is described herein. The "substantially pure" compound of the present disclosure herein is also used as a part of the present disclosure.

All configurational isomers of the compound of the present disclosure, whether in the form of mixtures, pure forms or highly pure forms, fall within the scope of the present disclosure. The definition of the compound of the present disclosure comprises both cis (Z) and trans (E) alkene isomers, and cis and trans isomers of carbocyclic and heterocyclic rings.

Throughout the specification, the groups and substituents may be selected to afford stable moieties and compounds.

Definitions of specific functional groups and chemical terms are described in detail hereinafter. For the present disclosure, chemical elements are defined in accordance with the Periodic Table of the Elements, CAS version, Handbook of Chemistry and Physics, 75th Ed. The definitions of the specific functional groups are also described therein. In addition, fundamental principles of organic chemistry, and the specific functional groups and reactivity thereof, are also described in the "Organic Chemistry", Thomas Sorrell, University Science Books, Sausalito: 1999, the entire contents of which are incorporated herein by reference.

Some compounds of the present disclosure may exist in specific geometric or stereoisomeric forms. The present disclosure comprises all the compounds, comprising cis and trans isomers, R and S enantiomers, diastereomers, (D) isomers, (L) isomers, racemic mixtures, and other mixtures thereof. In addition, the asymmetric carbon atoms may represent substituents, such as alkyl. All isomers and mixtures thereof are included within the present disclosure.

According to the present disclosure, mixtures of isomers may contain the isomers in various ratios. For example, a mixture containing only two isomers may be formed in a ratio as follows: 50: 50, 60: 40, 70: 30, 80: 20, 90: 10, 95: 5, 96: 4, 97: 3, 98: 2, 99: 1, or 100: 0, and all ratios of the isomers fall within the scope of the present disclosure. Similar ratios readily understandable to those skilled in the art and ratios of more complex isomeric mixtures also fall within the scope of the present disclosure.

The present disclosure further comprises an isotopically labeled compound equivalent to the originally disclosed compound herein. In practice, one or more atoms may be substituted with atoms having a different atomic mass or mass number. Examples of isotopes suitable for incorporation into the compound of the present disclosure comprise isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl respectively. The compound of the present disclosure, or the enantiomer, diastereomer, isomer, or pharmaceutically acceptable salt or solvate thereof, which contains the aforementioned isotopes of the compound or other isotopic atoms, falls within the scope of the present disclosure. Some isotopically labeled compounds of the present disclosure, comprising those labeled with radioactive isotopes ³H and ¹⁴C, are useful in tissue distribution experiments of drugs and substrates. Tritium, namely ³H, and carbon-14, namely ¹⁴C, are easy to prepare and detect, and are the preferred isotopes. Furthermore, substitution with heavier isotopes, such as deuterium, namely ²H, offers advantages in some therapeutic methods due to favorable metabolic stability, such as prolonging the in-vivo half-life or reducing dosage, and thus may be preferred under certain circumstances. The isotopically labeled compound may be prepared by conventional methods, wherein non-isotopic reagents are substituted with commercially available isotopically labeled reagents, following the procedures disclosed in the examples.

If it is necessary to design the synthesis of a specific enantiomer of the compound of the present disclosure, the enantiomer may be prepared by asymmetric synthesis, or by derivatization with a chiral auxiliary, separation of the resulting diastereomeric mixture, and subsequent removal of the chiral auxiliary to obtain the pure enantiomer. In addition, if a molecule contains a basic functional group, for example, an amino acid or acidic functional group, such as carboxyl, a diastereomeric salt may be formed with appropriate optically active acid or base, and then separated by conventional means such as separation and crystallization or chromatography to obtain the pure enantiomer.

As described herein, the compound of the present disclosure may be substituted with any number of substituents or functional groups to extend the scope of inclusion. Generally, the term "substituted", whether appearing before or after the term "optionally", within the general formula of substituents in the composition of the present disclosure, means that a hydrogen radical is substituted with a specified structural substituent. When multiple positions in a specific structure are substituted with multiple specific substituents, the substituent at each position may be identical or different. The term "substituted" as used herein comprises all permissible substitutions for organic compounds. In a broad sense, permissible substituents comprise acyclic, cyclic, branched, unbranched, carbocyclic, heterocyclic, aromatic and non-aromatic organic compounds. In the present disclosure, a heteroatom such as nitrogen may have a hydrogen substituent or any permissible organic compound described above to complete its valence. Furthermore, the present disclosure is not intended to limit the permissible substituted organic compounds in any way. It is contemplated by the present disclosure that combinations of substituents and variable groups are preferred where they form stable compounds useful for the treatment of diseases. The term "stable" as used herein refers to that the compound is stable enough to maintain the integrity of structure of the compound for a period sufficient for detection, and preferably remains effective for a sufficiently long period, as used herein for the purposes described above.

Metabolites of the compound in the present disclosure and the pharmaceutically acceptable salt thereof, and prodrugs that may be converted in vivo into structures of the compound in the present disclosure and the pharmaceutically acceptable salt thereof, are also included within the claims of the present disclosure.

In another preferred embodiment, any one of groups in the compound is respectively a corresponding group in the specific compound.

### Preparation method

A preparation method for a compound as represented by formula I is described in the following schemes and examples. Raw materials and intermediates are either purchased from commercial sources, prepared according to known procedures, or otherwise specified. In some cases, an order of steps for executing a reaction scheme may be changed to facilitate the reaction or to avoid unwanted by-products.

The preparation method for the compound as represented by formula I of the present disclosure is described in more detail hereinafter, and these specific methods shall not be construed as limiting the present disclosure in any respect. The compound of the present disclosure may also be conveniently prepared by optionally combining various synthetic methods described in the specification or known in the art, and such combinations may be readily performed by those skilled in the art to which the present disclosure belongs.

Generally, in the preparation processes, each reaction is ordinarily performed in a suitable solvent under the protection of an inert gas.

### Pharmaceutical composition and administration method

The pharmaceutical composition of the present disclosure is used for the prevention and/or treatment of neurodegenerative diseases (Alzheimer's disease, Parkinson's disease, progressive multiple sclerosis, amyotrophic lateral sclerosis, and the like), and is promising for use in the manufacture of a medicament for the treatment of a disease selected from the group consisting of cancer, progressive supranuclear palsy, mast cell activation syndrome, rheumatoid arthritis, asthma, Crohn's disease, sickle cell disease, and coronavirus disease 2019; wherein the cancer is selected from the group consisting of prostate cancer, pancreatic cancer, gastric cancer, liver cancer, multiple myeloma, and colorectal cancer.

The compound as shown in formula I may be used in combination with other known drugs for the treatment or amelioration of similar symptoms. In combined administration, administration method and dosage of an original drug may remain unchanged, and the compound as shown in formula I may be administered simultaneously or sequentially. When the compound as shown in formula I is co-administered with one or more other drugs, a pharmaceutical composition containing both the compound as shown formula I and one or more known drugs are preferably employed. The drug co-administration also comprises administration of the compound as shown in formula I and one or more other known drugs during an overlapping time period. When the compound as shown in formula I is administered in combination with one or more other drugs, a dose of the compound as shown in formula I or the known drug may be lower than that of the compound or the drug administered separately.

Dosage forms of the pharmaceutical composition of the present disclosure comprise, but are not limited to: an injection, a tablet, a capsule, an aerosol, a suppository, a film, a dripping pill, an external liniment, and a controlled-release agent a sustained-release agent or a nano-formulation.

The pharmaceutical composition of the present disclosure comprises a safe and effective amount of the compound of the present disclosure or the pharmacologically acceptable salt thereof, and the pharmacologically acceptable excipient or carrier. The "safe and effective amount" used herein means an amount of the compound sufficient to significantly ameliorate the condition without causing severe side effects. Generally, the pharmaceutical composition contains 1-2000 mg of the compound of the present disclosure per dose, and more preferably 10-1000 mg of the compound of the present disclosure per dose. Preferably, the "one dose" refers to one capsule or tablet.

"Pharmaceutically acceptable carrier" refers to one or more compatible solid or liquid fillers or gel substances, which are suitable for human use, and must have sufficiently high purity and sufficiently low toxicity. "Compatible" used herein means that components of the composition may be blended with the compound of the present disclosure and they do not significantly reducing the pharmacological efficacy of the compound when they are blended. Some examples of the pharmaceutically acceptable carrier comprise cellulose and a derivative thereof (such as sodium carboxymethyl cellulose, sodium ethyl cellulose and cellulose acetate), gelatin, talc, a solid lubricant (such as a stearic acid and magnesium stearate), calcium sulfate, a vegetable oil (such as soybean oil, sesame oil, peanut oil and olive oil), a polyol (such as propylene glycol, glycerin, mannitol and sorbitol), an emulsifier (such as Tween^{®}), a wetting agent (such as sodium dodecyl sulfate), a colorant, a flavoring agent, a stabilizer, an antioxidant, a preservative, pyrogen-free water, and the like.

An administration mode of the compound of the present disclosure or the pharmaceutical composition is not particularly limited, and representative administration modes comprise (but are not limited to): oral administration, intratumoral administration, rectal administration, parenteral (intravenous, intramuscular or subcutaneous) administration and topical administration.

Solid dosage forms for the oral administration comprise a capsule, a tablet, a pill, a powder and a granule. In these solid dosage forms, an active compound is mixed with at least one conventional inert excipient (or carrier), such as sodium citrate or dicalcium phosphate, or with the following components: (a) a filler or an extender, such as starch, lactose, sucrose, glucose, mannitol and silicic acid; (b) a binder, such as carboxymethylcellulose, alginates, gelatin, polyvinylpyrrolidone, sucrose and acacia; (c) a humectant, such as glycerol; (d) a disintegrant, such as agar, calcium carbonate, potato starch or tapioca starch, alginic acid, a certain complex silicate and sodium carbonate; (e) a solution retarding agent, such as paraffin; (f) an absorption accelerator, such as a quaternary ammonium compound; (g) a wetting agent, such as cetyl alcohol and glyceryl monostearate; (h) an adsorbent, such as kaolin; and (i) a lubricant, such as talc, calcium stearate, magnesium stearate, a solid polyethylene glycol, sodium lauryl sulfate, or a mixture of these substances. In the capsule, the tablet or the pill, the dosage forms may also comprise a buffering agent.

The solid dosage forms such as the tablet, the dragee, the capsule, the pill and the granule may be prepared with a coating and a shell, such as an enteric coating and other materials well known in the art, and may contain an opacifying agent. Moreover, the release of the active compound or the compound in such composition may be delayed to occur in a certain part of the digestive tract. Examples of suitable encapsulating components that can be used comprise a polymer substance and a wax substance. If necessary, the active compound may also form a microcapsule with one or more of the above excipients.

Liquid dosage forms for oral administration comprise a pharmaceutically acceptable emulsion, a solution, a suspension, a syrup and a tincture. In addition to the active compound, the liquid dosage forms may contain an inert diluent conventionally used in the art, such as water or other solvents, a solubilizer and an emulsifier, for example, ethanol, isopropanol, ethyl carbonate, ethyl acetate, propylene glycol, 1,3-butanediol, dimethylformamide and an oil, especially cottonseed oil, peanut oil, corn oil, olive oil, castor oil and sesame oil, or a mixture of these substances.

In addition to these inert diluents, the composition may also contain an adjuvant, such as a wetting agent, an emulsifying agent and a suspending agent, a sweetening agent, a flavoring agent and a perfume.

In addition to the active compound, the suspension may contain a suspending agent, for example, ethoxylated isostearyl alcohol, polyoxyethylene sorbitol and sorbitan ester, microcrystalline cellulose, aluminum methoxide and agar, or a mixture of these substances.

The composition for parenteral injection may comrpise a physiologically acceptable sterile aqueous or non-aqueous solution, dispersion, suspension or emulsion, and a sterile powder for redissolved into sterile injectable solution or dispersion. Suitable aqueous and non-aqueous carrier, diluent, solvent or excipient comprises water, ethanol, a polyol and a suitable mixture of these substances.

Dosage forms of the compound of the present disclosure for topical administration comprise an ointment, a powder, a patch, a spray and an inhalant. The active ingredient is mixed with a physiologically acceptable carrier and any preservative and buffer, or a propellant that may be required if necessary under sterile conditions

The therapeutic method of the present disclosure may be applied alone or in combination with other therapeutic means or therapeutic drugs.

When the pharmaceutical composition is applied, a safe and effective amount of the compound of the present disclosure is administered to a mammal (such as a human) in need of the treatment, wherein an administration dose is a pharmaceutically effective dose. For a human weighing 60 kg, a daily dose is generally 1-2000 mg, preferably 50-1000 mg. Certainly, a specific dose should also take into account an administration route, the health condition of a patient, and other factors, which are within the skill range of a skilled physician.

The present disclosure further provides a preparation method for the pharmaceutical composition, which comprises the step of mixing a pharmaceutically acceptable carrier with the compound as shown in formula I of the present disclosure, or the crystal form, pharmaceutically acceptable salt, hydrate or solvate thereof, so as to form the pharmaceutical composition.

The present disclosure further provides a therapeutic method, which comprises the step of administering to a subject in need of the treatment the compound as shown in formula I in the present disclosure, or the crystal form, pharmaceutically acceptable salt, hydrate or solvate thereof, or the pharmaceutical composition in the present disclosure, for the treatment or prevention of neurodegenerative diseases.

The present disclosure is further explained hereinafter with reference to specific embodiments. It should be understood that these embodiments are only used for explaining the present disclosure and are not intended to limit the scope of the present disclosure. Experimental methods for which specific conditions are not indicated in the following embodiments are generally carried out under conventional conditions, such as those described in Molecular Cloning: A Laboratory Manual, Sambrook et al. (New York: Cold Spring Harbor Laboratory Press, 1989), or under conditions recommended by the manufacturers. Unless otherwise stated, percentages and parts are calculated by weight.

Unless otherwise defined, all professional and scientific terms used herein have the same meanings well known to those skilled in the art. In addition, any methods and materials similar or equivalent to those described herein may be used in the method of the present disclosure. The preferred implementing methods and materials described herein are for illustrative purposes only.

The technical solution of the present disclosure is further described hereinafter, but the scope of protection of the present disclosure is not limited to this.

### Example 1

A structural formula of a compound provided in the example was as follows:

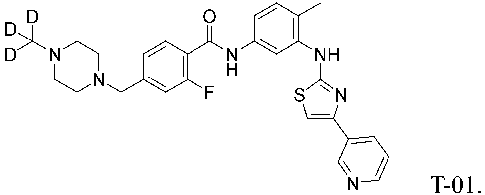

A specific synthetic process of the above compound T-01 was as follows:

### 1. Synthesis of compound SM1

### Synthetic route:

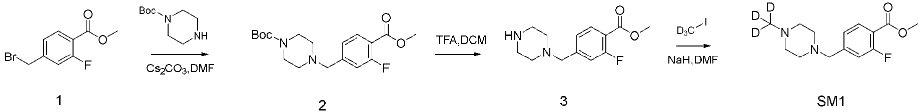

### Synthetic steps were as follows:

### S1: synthesis of compound 2

N-Boc-piperazine (4.5 g) and cesium carbonate (10.6 g) were added into a 100 mL three-necked flask, and then 50 mL of N,N-dimethylformamide was added for dissolution. After stirring at room temperature for 30 minutes, compound 1 (5.0 g) was added, and the reaction was continued at room temperature for 5 hours. When TLC analysis showed complete consumption of raw materials, the reaction was ended. The reaction solution was diluted with ethyl acetate, washed with water and a saturated sodium chloride solution sequentially, and dried with anhydrous sodium sulfate. The organic phase was concentrated directly, and separated and purified by silica gel column chromatography to obtain 6.8 g of compound 2.

### S2: synthesis of compound 3

Compound 2 (5.0 g) was added into a 100 mL three-necked flask, 50 mL of dichloromethane was added for stirred dissolution, and trifluoroacetic acid (8.5 g) was added dropwise in an ice bath. The reaction was performed at room temperature for 3 hours. When TLC analysis showed complete consumption of raw materials, the reaction was ended. A saturated sodium bicarbonate solution was added in an ice bath to adjust pH to 7.5, and then the mixture was extracted with ethyl acetate. The organic phase was washed with saturated sodium chloride, and dried with anhydrous sodium sulfate. The organic phase was directly concentrated without purification to obtain 3.4 g of compound 3, LCMS: [M+H]⁺=253.1.

### S3: synthesis of compound SM1

Compound 3 (3.4 g) was added into a 100 mL three-necked flask. After the system was subjected to nitrogen purging for three times, 34 mL of ultra-dry N,N-dimethylformamide was added under nitrogen protection for dissolution. The mixture was stirred in an ice bath, and sodium hydride (0.7 g) was added in portions. After the addition was completed, the mixture was stirred for 30 minutes, and then deuterated iodomethane (3.2 g) was added. The reaction was performed at room temperature for 3 hours. When TLC analysis showed complete consumption of raw materials, the reaction was ended. Water was added to the reaction solution, and then the mixture was extracted with ethyl acetate. The organic phase was washed with water and a saturated sodium chloride solution sequentially, and dried with anhydrous sodium sulfate. The organic phase was concentrated directly, and separated and purified by silica gel column chromatography to obtain 2.5 g of compound SM1.

### 2. Synthesis of compound SM3

### Synthetic route:

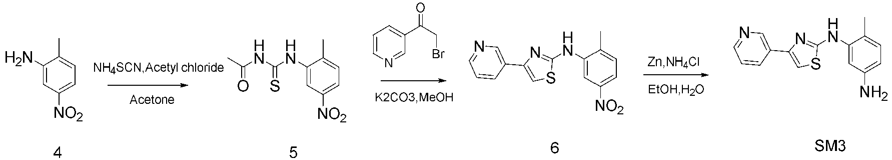

### Synthetic steps were as follows:

### S4: synthesis of compound 5

Compound 4 (10.0 g) was added into a 100 mL three-necked flask, 150 mL of acetone was added for dissolution, and then ammonium thiocyanate (6.0 g) and acetyl chloride (5.6 mL) were added. The reaction was performed at room temperature under nitrogen protection overnight. When TLC analysis showed complete consumption of raw materials, the reaction was ended. Water was added to quench the reaction. Suction filtration was performed, and the filter cake was washed with water and methyl tert-butyl ether sequentially, then dissolved with ethyl acetate, and separated and purified by silica gel column chromatography to obtain 8.8 g of compound 5, LCMS: [M+H]⁺=254.1.

### S5: synthesis of compound 6

Compound 5 (8.8 g) was added into a 100 mL three-necked flask, 90 mL of methanol was added for dissolution, and then potassium carbonate (14.4 g) and 3-(2-bromoacetyl)pyridine hydrobromide (9.8 g) were added sequentially. The reaction was performed at room temperature under nitrogen protection for 4 hours. When TLC analysis showed complete consumption of raw materials, the reaction was ended. Water was added to quench the reaction. Suction filtration was performed, and the filter cake was washed with water and methyl tert-butyl ether sequentially, and then dried at 60°C to obtain 9.5 g of compound 6, LCMS: [M+H]⁺=313.1.

### S6: synthesis of compound SM3

Compound 6 (9.0 g) was added into a 100 mL three-necked flask, 135 mL of ethanol/water mixed solvent (5: 1, V: V) was added for dissolution, and then ammonium chloride (4.6 g) and zinc powder (18.7 g) were added. After the system was subjected to nitrogen purging for three times, the reaction was performed at 80°C under nitrogen protection for 5 hours. When TLC analysis showed complete consumption of raw materials, the reaction was ended. Suction filtration was performed, ethanol was removed by evaporation, water was added to the residue, and the mixture was extracted with ethyl acetate. The organic phase was washed with saturated sodium chloride solution, and dried with anhydrous sodium sulfate. The organic phase was concentrated directly to obtain 6.6 g of compound SM3, LCMS: [M+H]⁺=283.1).

### 3. Synthesis of compound T-01

### Synthetic route:

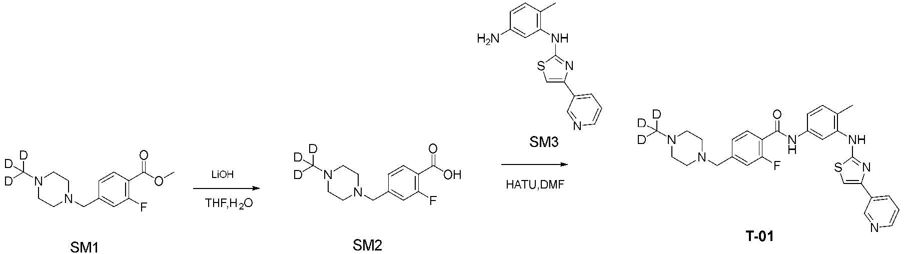

### Synthetic steps were as follows:

### S7: synthesis of compound SM2

Compound SM1 (500 mg) was added into a 50 mL three-necked flask, 10 mL of tetrahydrofuran was added for dissolution, and then 2 mL of aqueous solution containing lithium hydroxide (144 mg) was dropwise added. The reaction was performed at room temperature for 6 hours. When TLC analysis showed complete consumption of raw materials, the reaction was ended. 0.5 M hydrochloric acid was added to adjust pH to 4.7, then water was added, and the mixture was extracted with ethyl acetate. The organic phase was washed with saturated sodium chloride solution, and dried with anhydrous sodium sulfate. The organic phase was directly concentrated to obtain 433 mg of compound SM2, LCMS: [M-H]⁻=254.1.

### S8: synthesis of compound T-01

Compound SM2 (430 mg) was added into a 50 mL three-necked flask, 5 mL of N,N-dimethylformamide was added for dissolution, and then N,N,N',N'-tetramethyl-O-(7-azabenzotriazol-1-yl)- uronium hexafluorophosphate (1.2 g) was added. After stirring at room temperature for 30 minutes, compound SM3 (560 mg) was added, and the reaction was performed at room temperature overnight. When TLC analysis showed complete consumption of raw materials, the reaction was ended. Water was added to the reaction solution, and then the mixture was extracted with ethyl acetate. The organic phase was washed with saturated sodium chloride solution, and dried with anhydrous sodium sulfate. The organic phase was concentrated directly, and separated and purified by silica gel column chromatography to obtain 700 mg of compound T-01, LCMS: [M+H]⁺= 520.1.

### Example 2

A structural formula of a compound provided in the example was as follows:

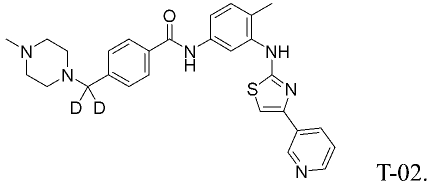

A specific synthetic process of the above compound T-02 was as follows:

### 1. Synthesis of compound SM4

### Synthetic route:

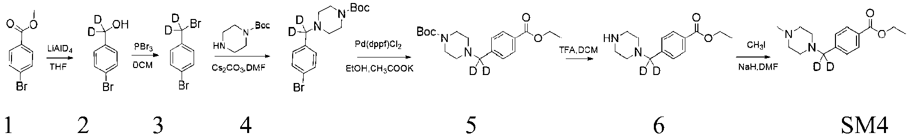

### Synthetic steps were as follows:

### S1: synthesis of compound 2

Compound 1 (5.0 g) was added into a 250 mL three-necked flask and dissolved with ultra-dry tetrahydrofuran (100 mL). Subsequently, the solution was cooled to 0°C under nitrogen protection, and lithium aluminum deuteride (976 mg) was added slowly. The reaction was performed under stirring at room temperature for 1 hour. When TLC analysis showed complete consumption of raw materials, the reaction was ended. The reaction solution was cooled to 0°C and then quenched with a saturated aqueous NH₄Cl solution. Insoluble solids were removed by suction filtration, water was added to the filtrate, and the mixture was extracted with ethyl acetate. The organic phase was washed with a saturated aqueous sodium chloride solution, dried with anhydrous sodium sulfate, and then concentrated to obtain 3.3 g of compound 2.

### S2: synthesis of compound 3

Compound 2 (3.3 g) was added into a 100 mL three-necked flask and dissolved with dichloromethane (30 mL). Subsequently, the solution was cooled to 0°C under nitrogen protection, and phosphorus tribromide (2.1 mL) was added slowly. The reaction was performed under stirring at room temperature for 3 hours. When TLC analysis showed complete consumption of raw materials, the reaction was ended. The reaction solution was cooled to 0°C and then quenched with a saturated aqueous sodium carbonate solution. The reaction solution was extracted with dichloromethane. The organic phase was washed with a saturated aqueous sodium chloride solution, dried with anhydrous sodium sulfate and then concentrated, and separated and purified by silica gel column chromatography to obtain 4.1 g of compound 3.

### S3: synthesis of compound 4

Compound 3 (4.1 g) was added into a 100 mL three-necked flask and dissolved with N,N-dimethylformamide (41 mL), and then N-Boc-piperazine (2.1 g) and cesium carbonate (7.9 g) were added sequentially. The reaction was performed under stirring at room temperature for 3 hours. When TLC analysis showed complete consumption of raw materials, the reaction was ended. Water was added to the reaction solution for quenching, and then the mixture was extracted with ethyl acetate. The organic phase was washed with a saturated aqueous NaCl solution, dried with anhydrous sodium sulfate and then concentrated, and separated and purified by silica gel column chromatography to obtain 5.2 g of compound 4.

### S4: synthesis of compound 5

Compound 4 (5.0 g) was added into a 500 mL reaction kettle and dissolved with absolute ethanol (150 mL), and then [1,1'-Bis(diphenylphosphino)ferrocene]dichloropalladium(II) (1.0 g) and potassium acetate (4.1 g) were added sequentially. After charging with CO to 0.6 MPa, the reaction was performed under heating and stirring at 100°C overnight. When TLC analysis showed complete consumption of raw materials, the reaction was ended. The reaction solution was cooled to room temperature. Insoluble solids were removed by suction filtration, the filtrate was concentrated to remove the solvent, then water was added, and the mixture was extracted with ethyl acetate. The organic phase was washed with a saturated aqueous sodium chloride solution, dried with anhydrous sodium sulfate and then concentrated, and separated and purified by silica gel column chromatography to obtain 3.8 g of compound 5.

### S5: synthesis of compound 6

Compound 5 (3.8 g) was added into a 100 mL three-necked flask and dissolved with dichloromethane (38 mL). Subsequently, the solution was cooled to 0°C, and trifluoroacetic acid (10.7 mL) was added slowly. The reaction was performed under stirring at room temperature for 5 hours. When TLC analysis showed complete consumption of raw materials, the reaction was ended. The reaction solution was cooled to 0°C, pH was adjusted to 7.6 with a saturated aqueous sodium bicarbonate solution, and then the reaction solution was extracted with dichloromethane. The organic phase was washed with a saturated aqueous sodium chloride solution, dried with anhydrous sodium sulfate, and then concentrated to obtain 2.7 g of compound 6.

### S6: synthesis of compound SM4

Compound 6 (2.7 g) was added into a 250 mL three-necked flask and dissolved with ultra-dry N,N-dimethylformamide (72 mL). The solution was cooled to 0°C under nitrogen protection, and sodium hydride (390 mg) was added slowly. The reaction was continued under stirring at 0°C for 0.5 hours. Subsequently, iodomethane (1.3 mL) was added, and then the reaction was performed under stirring at room temperature for 3 hours. When TLC analysis showed complete consumption of raw materials, the reaction was ended. Water was added to the reaction solution, and then the mixture was extracted with ethyl acetate. The organic phase was washed with a saturated aqueous sodium chloride solution, dried with anhydrous sodium sulfate and then concentrated, and separated and purified by silica gel column chromatography to obtain 2.3 g of compound SM4.

### 2. Synthesis of compound T-02

### Synthetic route:

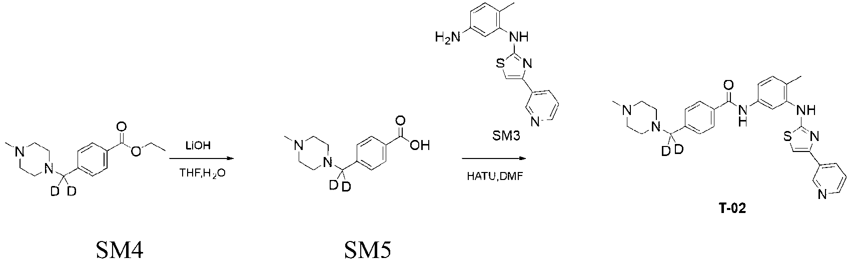

### Synthetic steps were as follows:

### S7: synthesis of compound SM5

The compound SM4 (1.0 g) was added into a 50 mL three-necked flask, 20 mL of tetrahydrofuran was added for dissolution, and then 4 mL of aqueous solution containing lithium hydroxide (300 mg) was dropwise added. The reaction was performed at room temperature for 6 hours. When TLC analysis showed complete consumption of raw materials, the reaction was ended. 0.5 M hydrochloric acid was added to the reaction solution to adjust pH to 4.3, then water was added, and the mixture was extracted with ethyl acetate. The organic phase was washed with saturated sodium chloride solution, and dried with anhydrous sodium sulfate. The organic phase was directly concentrated to obtain 883 mg of compound SM5, LCMS: [M-H]⁻=235.2.

### S8: synthesis of compound T-02

Compound SM5 (600 mg) was added into a 50 mL three-necked flask, 5 mL of N,N-dimethylformamide was added for dissolution, and then O-(7-azabenzotriazol-1-yl)-N,N,N',N'-tetramethyluronium hexafluorophosphate (1.7 g) was added. After stirring at room temperature for 30 minutes, compound SM3 (790 mg) was added, and the reaction was performed at room temperature overnight. When TLC analysis showed complete consumption of raw materials, the reaction was ended. Water was added to the reaction solution, and the mixture was extracted with ethyl acetate. The organic phase was washed with saturated sodium chloride solution, and dried with anhydrous sodium sulfate. The organic phase was concentrated directly, and separated and purified by silica gel column chromatography to obtain 867 mg of compound T-02, LCMS: [M+H]⁺=501.2.

### Example 3

A structural formula of a compound provided in the example was as follows:

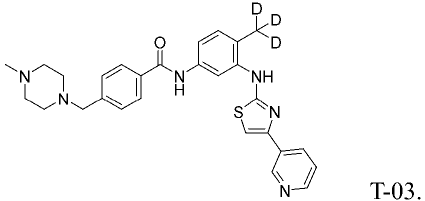

A specific synthetic process of the above compound T-03 was as follows:

### 1. Synthesis of compound SM6

### Synthetic route:

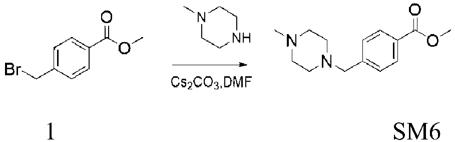

### Synthetic steps were as follows:

Compound 1 (2.0 g) was added into a 100 mL three-necked flask and dissolved with N,N-dimethylformamide (20 mL), and then N-methylpiperazine (0.96 g) and cesium carbonate (4.2 g) were added sequentially. The reaction was performed under stirring at room temperature for 3 hours. When TLC analysis showed complete consumption of raw materials, the reaction was ended. Water was added to the reaction solution for quenching, and then the mixture was extracted with ethyl acetate. The organic phase was washed with a saturated aqueous sodium chloride solution, dried with anhydrous sodium sulfate and then concentrated, and separated and purified by silica gel column chromatography to obtain 2.0 g of compound SM6.

### 2. Synthesis of compound SM8

### Synthetic route:

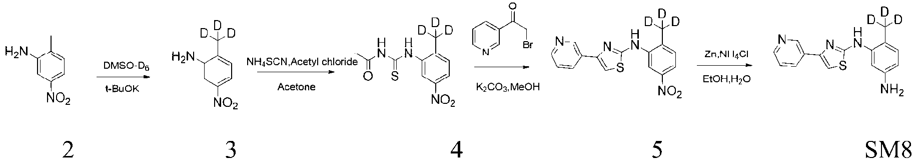

### Synthetic steps were as follows:

### S1: synthesis of compound 3

Compound 2 (5.0 g) was added into a 100 mL three-necked flask, deuterated dimethyl sulfoxide (50 mL) was added for dissolution, then potassium tert-butoxide (5.5 g) was added, and deuterium oxide (20 mL) was added. The reaction was performed at room temperature under nitrogen protection overnight. Water was directly added to the reaction solution, and the mixture was extracted with ethyl acetate. The organic phase was washed with a saturated aqueous sodium chloride solution, dried with anhydrous sodium sulfate, and then concentrated to obtain 4.89 g of compound 3.

### S2: synthesis of compound 4

Compound 3 (4.8 g) was added into a 100 mL three-necked flask, 75 mL of acetone was added for dissolution, and then ammonium thiocyanate (2.9 g) and acetyl chloride (2.7 mL) were added. The reaction was performed at room temperature under nitrogen protection overnight. When TLC analysis showed complete consumption of raw materials, the reaction was ended. Water was added to quench the reaction. Suction filtration was performed, and the filter cake was washed with water and methyl tert-butyl ether sequentially, then dissolved with ethyl acetate, and separated and purified by silica gel column chromatography to obtain 3.8 g of compound 4, LCMS: [M+H]⁺=257.1.

### S3: synthesis of compound 5

Compound 4 (3.8 g) was added into a 100 mL three-necked flask, 40 mL of methanol was added for dissolution, and then potassium carbonate (6.1 g) and 3-(2-bromoacetyl)pyridine hydrobromide (4.1 g) were added sequentially. The reaction was performed at room temperature under nitrogen protection for 4 hours. When TLC analysis showed complete consumption of raw materials, the reaction was ended. Water was added to quench the reaction. Suction filtration was performed, and the filter cake was washed with water and methyl tert-butyl ether sequentially, and then dried at 60°C to obtain 3.9 g of compound 5, LCMS: [M+H]⁺=316.1.

### S4: synthesis of compound SM8

Compound 5 (3.9 g) was added into a 100 mL three-necked flask, 60 mL of ethanol and water mixed solvent (5: 1, V: V) was added for dissolution, and then ammonium chloride (2.0 g) and zinc powder (8.0 g) were added. After the system was subjected to nitrogen purging for three times, the reaction was performed at 80°C under nitrogen protection for 5 hours. When TLC analysis showed complete consumption of raw materials, the reaction was ended. Suction filtration was performed, ethanol was removed by evaporation, water was added to the residue, and the mixture was extracted with ethyl acetate. The organic phase was washed with saturated sodium chloride solution, and dried with anhydrous sodium sulfate. The organic phase was concentrated directly to obtain 3.0 g of compound SM8, LCMS: [M+H]⁺=286.1.

### 3. Synthesis of compound T-03

### Synthetic route:

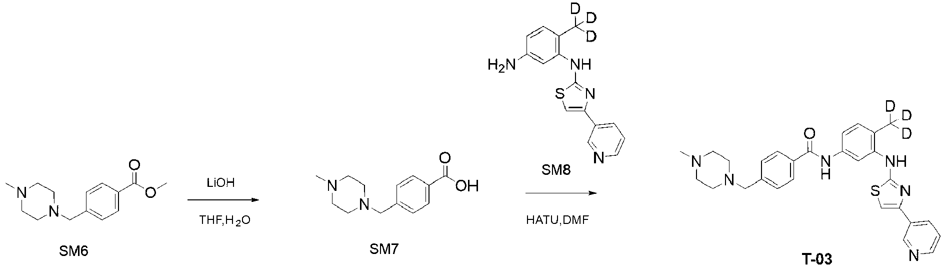

### Synthetic steps were as follows:

### S5: synthesis of compound SM7

The compound SM6 (1.0 g) was added into a 50 mL three-necked flask, 20 mL of tetrahydrofuran was added for dissolution, and then 4 mL of aqueous solution containing lithium hydroxide (300 mg) was dropwise added. The reaction was performed at room temperature for 6 hours. When TLC analysis showed complete consumption of raw materials, the reaction was ended. 0.5 M hydrochloric acid was added to adjust pH to 4.8, water was added, and the mixture was extracted with ethyl acetate. The organic phase was washed with saturated sodium chloride solution, and dried with anhydrous sodium sulfate. The organic phase was directly concentrated to obtain 860 mg of compound SM7, LCMS: [M-H]⁻=233.2.

### S6: synthesis of compound T-03

Compound SM7 (850mg) was added into a 50 mL three-necked flask, 5 mL of N,N-dimethylformamide was added for dissolution, and then O-(7-azabenzotriazol-1-yl)-N,N,N',N'-te-tramethyluronium hexafluorophosphate (2.5g) was added. After stirring at room temperature for 30 minutes, compound SM8 (1.1g) was added, and the reaction was performed at room temperature overnight. When TLC analysis showed complete consumption of raw materials, the reaction was ended. Water was added to the reaction solution, and the mixture was extracted with ethyl acetate. The organic phase was washed with saturated sodium chloride solution, and dried with anhydrous sodium sulfate. The organic phase was concentrated directly, and separated and purified by silica gel column chromatography to obtain 1.3 g of compound T-03, LCMS: [M+H]⁺=502.2.

### Synthesis of partial intermediates:

### Synthesis of intermediate 1

### Synthetic route:

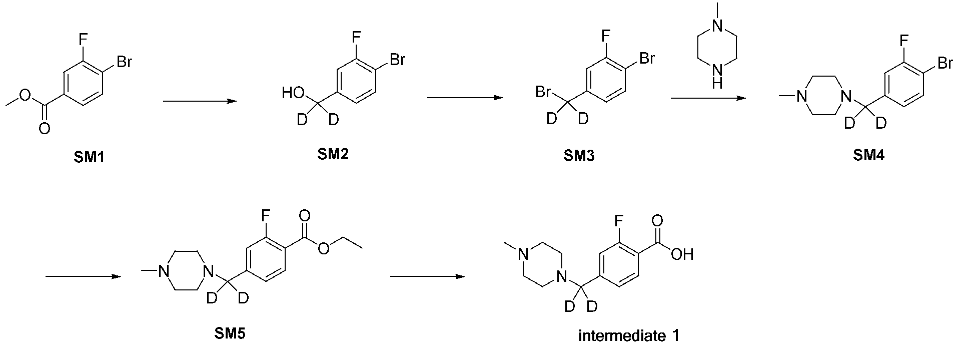

### Experimental process:

### Synthesis of SM2

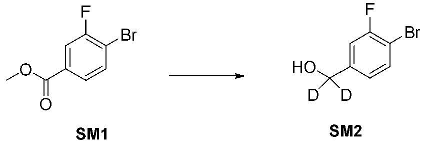

Compound SM1 (6 g, 1.0 eq) was dissolved in THF (60 mL), the system was subjected to nitrogen purging for three times, and LiAlD₄ (1.08 g, 1.0 eq) was added in portions in an ice bath (0°C). The mixture was recovered to room temperature, the reaction was performed at room temperature for 3 hours, and product formation was monitored by TLC. Post-treatment: saturated NH₄Cl was added for quenching in an ice bath, a part of THF was removed by rotary evaporation, and then H₂O and EA were added. Suction filtration was performed through diatomaceous earth, and then an organic layer was collected and evaporated to dryness to obtain the SM2 (4.4 g).

### Synthesis of SM3

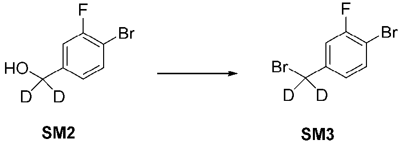

SM2 (44.3 g, 1.0 eq) was dissolved in DCM (45 mL), the system was subjected to nitrogen purging for three times, and PBr₃ (2.02 mL, 1.0 eq) was added in an ice bath (0°C). The mixture was recovered to room temperature, the reaction was performed at room temperature for 3 hours, and product formation was monitored by TLC. Post-treatment: saturated NaHCO₃ was added in an ice bath to adjust pH to approximately 8, most of DCM was removed by rotary evaporation, and then H₂O and EA were added for extraction. The organic phase was evaporated to dryness to obtain the SM3 (3.6 g).

### Synthesis of SM4

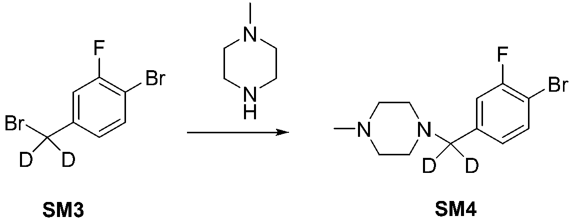

Compound SM3 (1.8 g, 1.0 eq), N-methylpiperazine (740 mg, 1.1 eq) and Cs₂CO₃ (3.3 g, 1.5 eq) were dissolved in DMF (18 mL), and the system was subjected to nitrogen purging for three times. The reaction was performed at room temperature overnight, and product formation was monitored by TLC. Post-treatment: H₂O and EA were added for extraction, and the EA layer was washed once with pure water and then washed once with saturated NaCl, and evaporated to dryness to obtain the SM4 (1.7 g).

### Synthesis of SM5

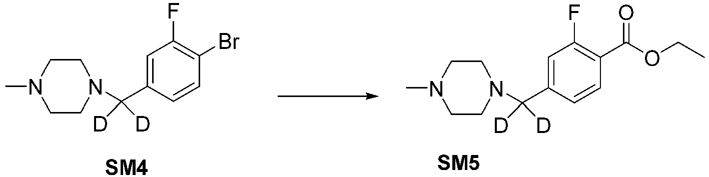

SM4 (1.7 g, 1.0 eq) was dissolved in EtOH (170 mL), and Pd(dppf)Cl₂ (432 mg, 0.1 eq) and AcOK (1.7 g, 3.0 eq) were added. CO replacement was performed. The system was heated to 100°C and the reaction was performed overnight, and product formation was monitored by TLC. Post-treatment: filtration was performed through diatomaceous earth, and the filtrate was evaporated to dryness and loaded with silica gel, and purified by column chromatography to obtain SM5 (1.46 g).

### Synthesis of intermediate 1

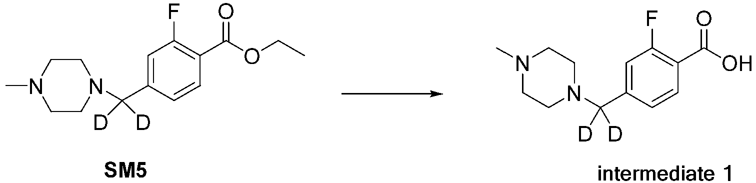

SM5 (1.46 g, 1.0 eq) was dissolved in THF (29.2 mL), and LiOH (732 mg, 3.0 eq), water (14.6 mL) and EtOH (4.9 mL) were added. The system was subjected to nitrogen purging for three times. The system was heated to 50°C and the reaction was performed overnight, and product formation was monitored by TLC. Post-treatment: filtration was performed through diatomaceous earth, most of THF was removed by rotary evaporation, and 1 N HCl was added to adjust pH to 2-3. Solid NaCl was continuously added to saturate the solution, and solids were produced after precipitation and filtered to obtain the filter cake intermediate 1 (1 g).

### Synthesis of intermediate 2

### Synthetic route:

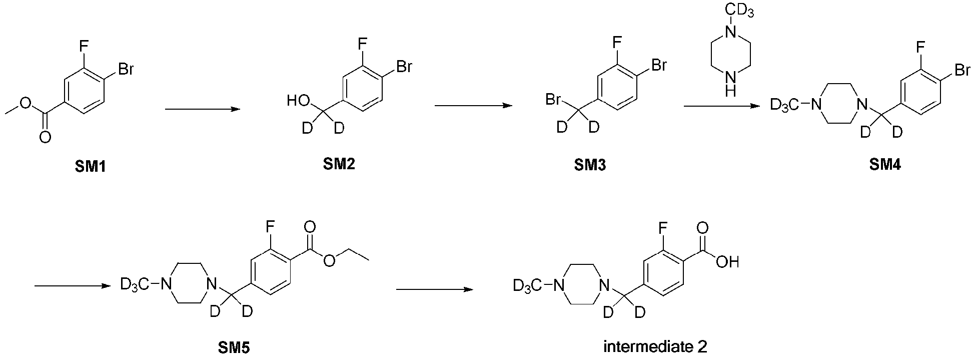

### Experimental process:

### Synthesis of SM2

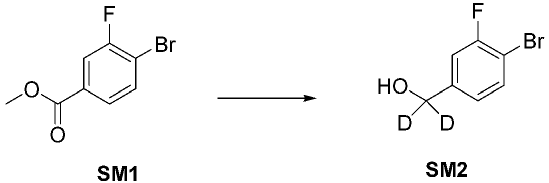

Compound SM1 (6 g, 1.0 eq) was dissolved in THF (60 mL), the system was subjected to nitrogen purging for three times, and LiAlD₄ (1.08 g, 1.0 eq) was added in portions in an ice bath (0°C). The mixture was recovered to room temperature, the reaction was performed at room temperature for 3 hours, and product formation was monitored by TLC. Post-treatment: saturated NH₄Cl was added for quenching in an ice bath, a part of THF was removed by rotary evaporation, and then H₂O and EA were added. Suction filtration was performed through diatomaceous earth, and then an organic layer was collected and evaporated to dryness to obtain SM2 (4.4 g).

### Synthesis of SM3

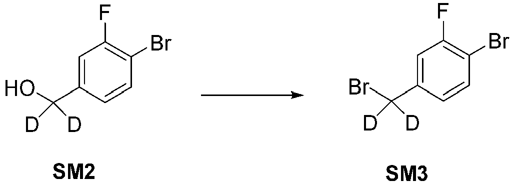

SM2 (44.3 g, 1.0 eq) was dissolved in DCM (45 mL), the system was subjected to nitrogen purging for three times, and PBr₃ (2.02 mL, 1.0 eq) was added in an ice bath (0°C). The mixture was recovered to room temperature, the reaction was performed at room temperature for 3 hours, and product formation was monitored by TLC. Post-treatment: saturated NaHCO₃ was added in an ice bath to adjust pH to approximately 8, most of DCM was removed by rotary evaporation, and then H₂O and EA were added for extraction. The organic phase was evaporated to dryness to obtain SM3 (3.6 g).

### Synthesis of SM4

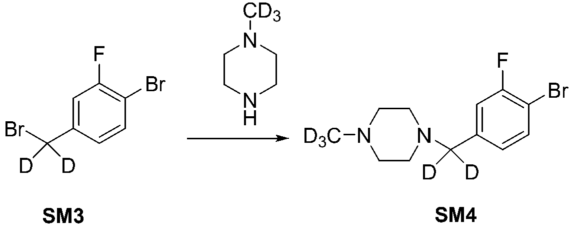

Compound SM3 (1.8 g, 1.0 eq), N-methylpiperazine (763 mg, 1.1 eq) and Cs₂CO₃ (3.3 g, 1.5 eq) were dissolved in DMF (18 mL), and the system was subjected to nitrogen purging for three times. The reaction was performed at room temperature overnight, and product formation was monitored by TLC. Post-treatment: H₂O and EA were added for extraction, and the EA layer was washed once with pure water and then washed once with saturated NaCl, and evaporated to dryness to obtain SM4 (1.27g).

### Synthesis of SM5

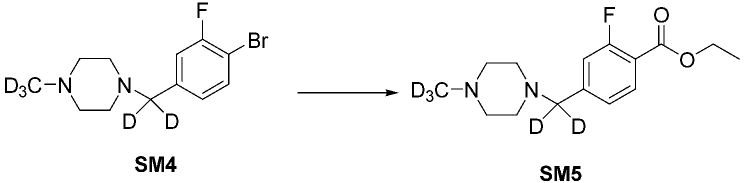

SM4 (1.27 g, 1.0 eq) was dissolved in EtOH (127 mL), and Pd(dppf)Cl₂ (319 mg, 0.1 eq) and AcOK (1.3 g, 3.0 eq) were added. CO replacement was performed. The system was heated to 100°C and the reaction was performed overnight, and product formation was monitored by TLC. Post-treatment: filtration was performed through diatomaceous earth, and the filtrate was evaporated to dryness and loaded with silica gel, and purified by column chromatography to obtain SM5 (350mg).

### Synthesis of intermediate 2

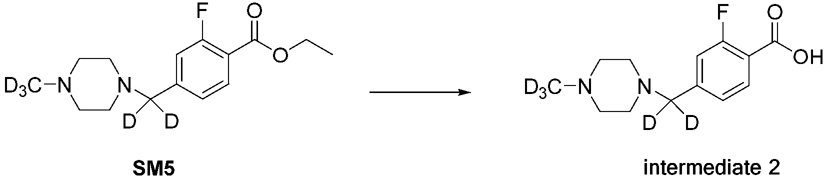

SM5 (350 mg, 1.0 eq) was dissolved in THF (7 mL), and LiOH (155 mg, 3.0 eq), water (3.5 mL) and EtOH (1.75 mL) were added. The system was subjected to nitrogen purging for three times. The system was heated to 50°C and the reaction was performed overnight, and product formation was monitored by TLC. Post-treatment: filtration was performed through diatomaceous earth, most of THF was removed by rotary evaporation, and 1 N HCl was added to adjust pH to 2-3. Solid NaCl was continuously added to saturate the solution, and solids were produced after precipitation and filtered to obtain the filter cake intermediate 2 (320 mg).

### Synthesis of intermediate 3

### Synthetic route:

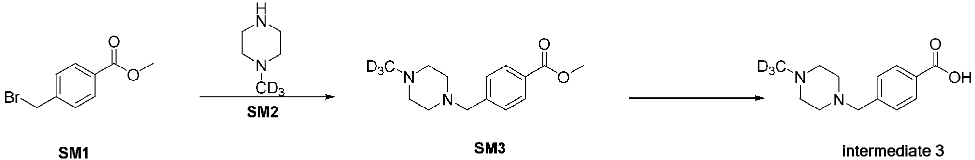

### Experimental process:

### Synthesis of SM3

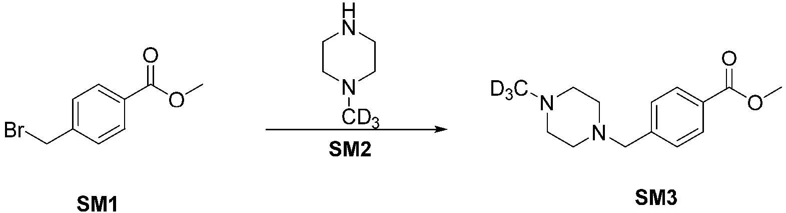

Compound SM1 (2.2 g, 1.0 eq), compound SM2 (1 g, 1.0 eq), Cs₂CO₃ (6.3 g, 2.0 eq), Xantphos (561 mg, 0.10 eq) and Pd(OAc)₂ (108 mg, 0.05 eq) were dissolved in ultra-dry 1,4-dioxane (22 mL). The system was subjected to nitrogen purging for three times. The system was heated to 105°C and the reaction was performed for 1 hour, and product formation was monitored by TLC. Post-treatment: dilution was performed with EA, filtration was performed through diatomaceous earth, and the filtrate was concentrated to dryness, then evaporated to dryness and loaded with silica gel, and purified by column chromatography to obtain SM3 (1.46 g).

### Synthesis of intermediate 3

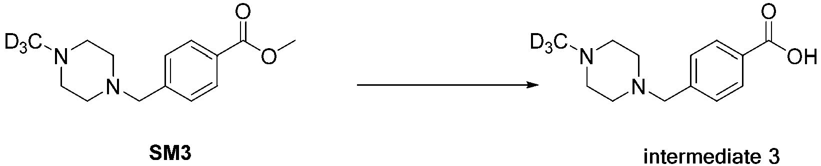

SM3 (1.46 g, 1.0 eq) was dissolved in THF (29.2 mL), and LiOH (732 mg, 3.0 eq), water (14.6 mL) and EtOH (4.9 mL) were added. The system was subjected to nitrogen purgingfor three times. The system was heated to 50°C and the reaction was performed overnight, and product formation was monitored by TLC. Post-treatment: filtration was performed through diatomaceous earth, most of THF was removed by rotary evaporation, and 1 N HCl was added to adjust pH to 2-3. Solid NaCl was continuously added to saturate the solution, and solids were produced after precipitation and filtered to obtain the filter cake intermediate 3 (1 g).

### Synthesis of intermediate 4

### Synthetic route:

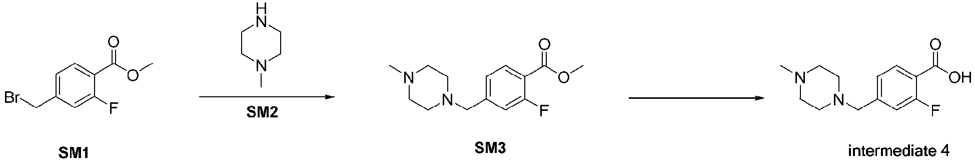

### Experimental process:

### Synthesis of SM3

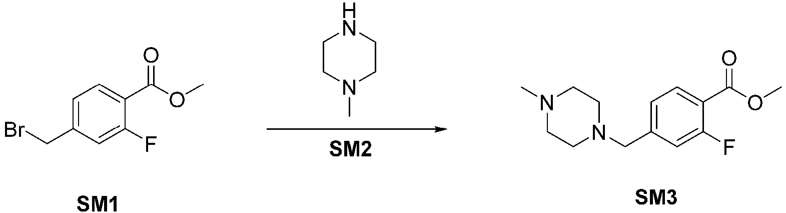

Compound SM1 (900 mg, 1.0 eq), compound SM2 (365 mg, 1.0 eq), Cs₂CO₃ (2.368 g, 2.0 eq), Xantphos (211 mg, 0.10 eq) and Pd(OAc)₂ (41 mg, 0.05 eq) were dissolved in ultra-dry 1,4-dioxane (9 mL). The system was subjected to nitrogen purging for three times. The system was heated to 105°C and the reaction was performed for 4 hours, and product formation was monitored by TLC. Post-treatment: dilution was performed with EA, filtration was performed through diatomaceous earth, and the filtrate was concentrated to dryness, then evaporated to dryness and loaded with silica gel, and purified by column chromatography to obtain SM3 (908g).

### Synthesis of intermediate 4

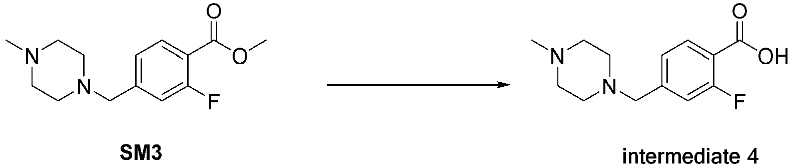

SM3 (908 mg, 1.0 eq) was dissolved in THF (36 mL), and LiOH (430 mg, 3.0 eq), water (9 mL) and EtOH (18 mL) were added. The system was subjected to nitrogen purging for three times. The system was heated to 50°C and the reaction was performed overnight, and product formation was monitored by TLC. Post-treatment: filtration was performed through diatomaceous earth, most of THF was removed by rotary evaporation, and 1 N HCl was added to adjust pH to 2-3. Solid NaCl was continuously added to saturate the solution, and solids were produced after precipitation and filtered to obtain the filter cake intermediate 4 (1.1g).

Commercially available raw materials and the above synthesized intermediates were used. With reference to the synthetic methods in the above examples, the specific compounds listed in Table 1 below were synthesized respectively.

**Table 1**

| Serial number of compound | Structure of compound | Characterization data |
|---|---|---|
| T-04 | 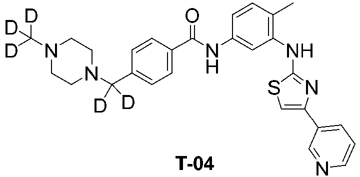 | LCMS: [ M + H ]⁺= 504.2 |
| T-05 | 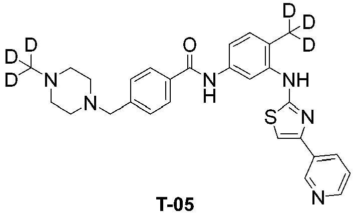 | LCMS: [ M + H ]⁺= 505.2 |
| T-06 | 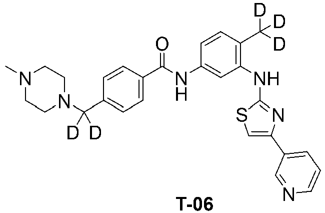 | LCMS: [ M + H ]⁺= 504.2 |
| T-07 | 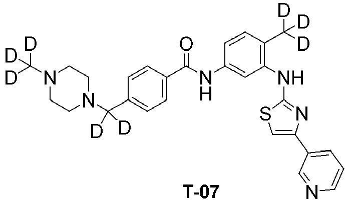 | LCMS: [ M + H ]⁺= 507.2 |
| T-08 | 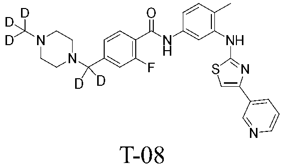 | LCMS: [ M + H ]⁺= 522.2 |
| T-09 | 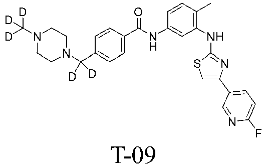 | LCMS: [ M + H ]⁺= 522.2 |
| T-11 | 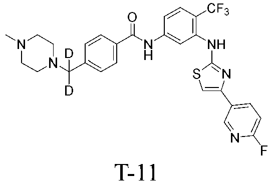 | LCMS: [ M + H ]⁺= 573.1 |
| T-12 | 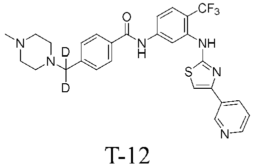 | LCMS: [ M + H ]⁺= 555.2 |
| T-14 | 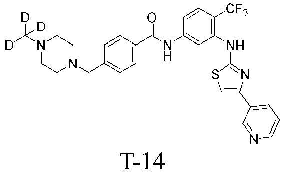 | LCMS: [ M + H ]⁺= 556.2 |
| T-15 | 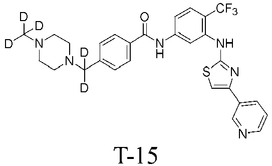 | LCMS: [ M + H ]⁺= 558.2 |
| T-16 | 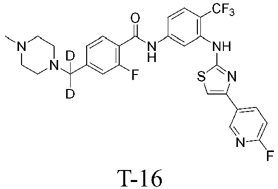 | LCMS: [ M + H ]⁺= 591.1 |
| T-17 | 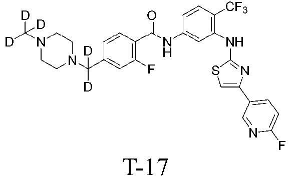 | LCMS: [ M + H ]⁺= 594.2 |
| T-18 | 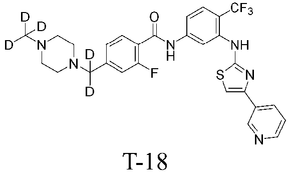 | LCMS: [ M + H ]⁺= 576.2 |
| T-19 | 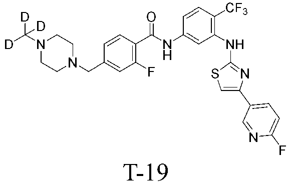 | LCMS: [ M + H ]⁺= 592.1 |
| T-20 | 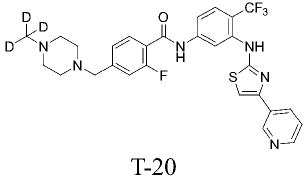 | LCMS: [ M + H ]⁺= 574.2 |
| T-21 | 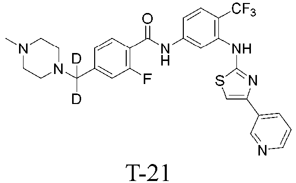 | LCMS: [ M + H ]⁺= 573.2 |

### Test Example 1 Enzyme activity assay

Biological activity tests were performed on partial compounds synthesized in the above examples and comparative examples below. Activities of the compounds toward c-Kit kinase were determined respectively, and results can be seen in Table 2 below.

**Table 2**

| Serial number of compound | c-Kit kinase activity (IC₅₀ nM) |
|---|---|
| T-01 | 1.67 |
| T-02 | 2.58 |
| T-04 | 2.07 |
| T-08 | 3.89 |
| T-11 | 3.55 |
| T-12 | 2.13 |
| T-15 | 3.56 |
| T-16 | 2.12 |
| T-17 | 1.35 |
| T-18 | 3.12 |
| T-19 | 2.11 |
| T-20 | 2.10 |
| T-21 | 1.36 |
| Control sample: Masitinib | 5.12 |

Structural formula of control sample Masitinib

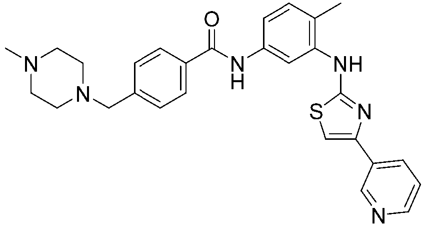

### Test Example 2 Preclinical pharmacokinetic test in rats

### I. Experimental materials and equipment

Healthy adult SD rats, male, aged 6-8 weeks, with a body weight of 200-300 g, purchased from Vital River Company; EDTA-Na2 anticoagulant; and analytical balance, animal weighing scale, magnetic stirrer, refrigerated centrifuge, single-channel manual pipette, and the like.

### II. Experimental process

1. Drug preparation: approximately 10 mg of the test sample was accurately weighed and dissolved in 5% DMSO (after conversion), and then 10% solutol HS-15 and 85% normal saline were added. The mixture was subjected to ultrasonic processing and mixed by vortexing to obtain a solution at a concentration of 1 mg/mL, which was freshly prepared before use.
   0.2 mL of the sample was transferred into a 1.5 mL centrifuge tube and stored at -80°C for administration solution concentration analysis.
2. Animal preparation: the rats were fed in rat cages, and fasted (for at least 10 hours) with free access to water one day before the experiment. On the day of experiment, the rats were weighed and marked at the tail respectively. Blank blood samples were collected respectively before drug administration. The blood samples were collected via the tail vein.
3. Drug administration:
   administration route: intragastric administration (p.o.);
   administration dose: 10 mg/kg;
   administration volume: 10 mL/kg; and
   operation procedure: the rat was restrained in an upright position with a left hand wearing bite-resistant gloves, a 16-gauge gavage needle was inserted through the oral cavity into the pharynx and advanced without obvious resistance, and then the drug was then injected into the stomach.
4. Sample collection: whole blood (0.1 mL) was collected from each test animal before drug administration and at 0.5 hours, 1 hour, 2 hours, 4 hours, 6 hours, 8 hours, 12 hours and 24 hours after drug administration, transferred into EDTA-Na2 anticoagulant tubes, mixed evenly by inverting the tubes 3-4 times, and then centrifuged at 10000 g and 4°C for 5 minutes to separate plasma. The obtained plasma samples were stored at -80°C for later analysis. The blood samples were collected via the tail vein.

Specific operation: the rat was secured in a restrainer to fully expose its tail, the tail was wiped with alcohol to make the surface skin absorb the alcohol, so as to induce obvious venous dilation. A suitable vein was selected from two sides, and the needle was inserted at approximately one-third of the distance from the tail tip. An insulin syringe was used as the injection syringe, the needle was inserted with the bevel facing upward, and the needle was immediately adjusted to a parallel position after piercing the skin. Slight sliding resistance of the needle inside the vessel and blood reflux in the injection syringe indicated successful venous access. Approximately 0.1-0.2 mL of whole blood was drawn. Gentle compression was applied for hemostasis after needle removal.

### III. Sample analysis

Preparation of standard curve sample: 25 µL of blank rat plasma was transferred into a centrifuge tube respectively, 25 µL of prepared serial standard solution (prepared in methanol) was added, and then 200 µL of internal standard solution (prepared in methanol) was added. The mixture was mixed by vortexing for 2 minutes, and centrifuged at 10000 g and 4°C for 10 minutes.

Treatment of the unknown plasma sample: 25 µL of drug-containing rat plasma was collected respectively, and then 25 µL of methanol and 200 µL of internal standard solution were added sequentially. The mixture was mixed by vortexing for 2 minutes, and centrifuged at 10000 g and 4°C for 10 minutes. The supernatant was collected for LC/MS/MS detection.

### IV. Data processing

A quantitative detection method for the test compound was established by using a Shimadzu liquid chromatography system and a Triple Quad TM 6500+AB mass spectrometer. A concentration of a parent drug in plasma was determined. Plasma concentration-time curves were plotted, and major pharmacokinetic parameters were calculated via a non-compartmental model in winnonlin Phoenix software. Pharmacokinetic results can be seen in Table 3 below:

**Table 3**

| Serial number of compound | Cmax (ng/mL) | AUC (h*ng/mL) |
|---|---|---|
| T-01 | 418.66 | 2687.12 |
| T-02 | 396.67 | 2368.17 |
| T-04 | 486.32 | 3259.13 |
| T-16 | 516.30 | 4012.56 |
| T-17 | 533.18 | 4289.78 |
| T-18 | 501.08 | 3998.14 |
| Control sample: Masitinib | 321.33 | 1771.48 |

### Test Example 3 Brain-penetrating pharmacokinetic test

### 1. Drug preparation

### Administration vehicle: 5% DMSO+10% Solutol HS15+85% normal saline

Formulation preparation process: an appropriate amount of powder was weighed, an appropriate volume of DMSO was added, and the mixture was mixed by vortexing. Subsequently, an appropriate volume of Solutol solution was added and vortexed, then normal saline was added, and the mixture was subjected to vortexing and ultrasonic processing to form a homogeneous and clear formulation solution.

Two formulation samples collected before drug administration and the remaining formulation after drug administration were stored at 2-8°C.

### 2. Experimental animals

The experimental animals were fed in an animal room. The animal room was equipped with an air-conditioning system, with adequate ventilation, an indoor temperature was maintained at 20-26°C, and a relative humidity was maintained at 40%-70%. Artificial lighting was applied in the animal room, with a 12-hour light/dark cycle (except for working lights turned on during experimental operations and cleaning). The experimental animals had free access to food and water.

The purchased animals were fed normally for at least 3 days, rats with healthy physical conditions were included in the experiment, and each rat was marked with a tail number. Animals in an oral administration group were fasted overnight one day before drug administration, and fed 4 hours after drug administration, with free access to water.

A source and a number of the animals used in the experiment can be seen in Table 4.

**Table 4**

| Species | Strain | Source | Body weight | Weeks of age (W) | Number of male animals |
|---|---|---|---|---|---|
| Rat | Sprague-Dawley (SD) | Vital River Laboratory Animal Technology Co., Ltd. | 180-220 g | 6-10 | 18 |

| | | | | | |
|---|---|---|---|---|---|
| Note: 3 rats per group. | | | | | |

### 3. Drug administration

Administration route: intragastric administration (p.o.)
Administration concentration: 1 mg/mL
Administration dose: 10 mg/kg
Administration volume: 10 mL/kg
Administration frequency: single administration

Before drug administration, a state of the administration formulation was examined, the homogeneity of the formulation was ensured by vortexing, stirring or shaking, and a theoretical administration volume for each SD rat in every group was calculated according to the following formula. $\begin{matrix}Theoretical \\ administration volume\end{matrix} \left(mL\right) = \frac{Dose \left(mg⋅{kg}^{- 1}\right)}{\begin{matrix}Formulation \\ concentration\end{matrix} \left(mg⋅{mL}^{- 1}\right)} \times \begin{matrix}Body \\ weight\end{matrix} \left(kg\right)$

### 4. Sample collection and processing

The rats were sampled at 1 hour, 2 hours, 4 hours, 6 hours, 8 hours and 24 hours after drug administration.

Plasma: at each time point, 0.25 mL of whole blood was collected from the jugular vein and transferred into a test tube containing an anticoagulant EDTA-K₂ (7 µL, 10% EDTA-K₂ solution), placed on wet ice, and centrifuged within 1 hour (2,000 g, 2-8°C, 10 minutes), and then, the plasma was taken. The plasma was stored in a pre-chilled centrifuge tube, rapidly frozen in dry ice, and then stored in an ultra-low temperature refrigerator at -60°C or lower until LC-MS/MS analysis.

Cerebrospinal fluid: the rats were euthanized via a carbon dioxide process, and approximately 60 µL of cerebrospinal fluid was collected by puncture. 1 mL syringe was used, with the bevel of the needle facing upward, the needle tip was inserted into the subarachnoid space in a nearly horizontal position, then the needle was fixed, and the cerebrospinal fluid was slowly aspirated. The collected samples were frozen on dry ice within 30 minutes, and then transferred to an environment at -90°C to -60°C.

Brain tissue: cardiac perfusion was performed first, and then the brain tissue was isolated. Under deep terminal anesthesia, approximately 80 mL of normal saline was used for cardiac perfusion, and residual blood in the brain tissue was flushed out of cerebral circulation. The isolated brain tissue was gently rinsed once with ice-cold normal saline, excess water was removed, weighed, and then transferred to a frozen storage environment at -90°C to -60°C.

### 5. Sample analysis and data processing

### 5.1 Sample analysis

A quantitative detection method for the test compound was established by using a Shimadzu liquid chromatography system and a Triple Quad TM 6500+AB mass spectrometer. A concentration of a parent drug in a sample was analyzed. Quality control samples were used for variation control over analysis results, and the accuracy of the quality control samples should fall within the range of 80%-120%.

### 5.2 Data processing

Pharmacokinetic parameters: Tmax, Cmax, AUC (0-t), AUC (0-∞), T1/2, MRT (0-∞), and the like were calculated by using winnonlin Phoenix 8.1.0.3530.

Pharmacokinetic data of the test samples obtained from the above analysis can be seen in Table 5 below.

**Table 5**

| Serial number of compound | Total drug concentration in brain tissue (h*ng/ml) | Total drug concentration in plasma (h*ng/ml) | Total drug brain-to-plasma ratio (B/P) |
|---|---|---|---|
| T-01 | 1370.43 | 2687.12 | 0.51 |
| T-08 | 1798.12 | 3269.32 | 0.55 |
| T-16 | 3129.79 | 4012.56 | 0.78 |
| T-20 | 3092.40 | 4123.20 | 0.75 |
| T-21 | 2763.22 | 3891.87 | 0.71 |
| Control sample: Masitinib | 356.98 | 1771.48 | 0.20 |

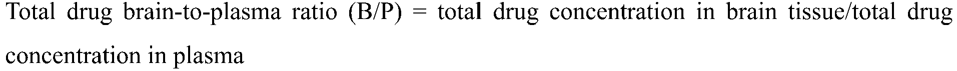

Total drug brain-to-plasma ratio (B/P) = total drug concentration in brain tissue/total drug concentration in plasma

Compared with the control sample Masitinib, our compound exhibited higher drug exposure and brain drug exposure. Therefore, the compound of the present disclosure had greater potential for the treatment of brain tumor, Alzheimer's disease, Parkinson's disease, amyotrophic lateral sclerosis, and the like.

All documents mentioned in the present disclosure are incorporated by reference in the present disclosure, just as each document is individually incorporated by reference. In addition, it should be understood that, after reading the above contents explained in the present disclosure, those skilled in the art can make various changes or modifications to the present disclosure, and these equivalent forms also fall within the scope defined by the appended claims of the present disclosure.

## Claims

1. A polycyclic compound, wherein the compound is a compound as represented by formula I, or a pharmaceutically acceptable salt, stereoisomer, tautomer, hydrate, solvate, isotopic compound or prodrug thereof: 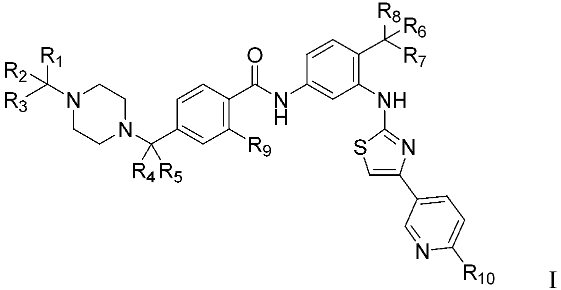 wherein:
R₁, R₂, R₃, R₄ and R₅ each independently represent H or D, and at least one of R₁, R₂, R₃, R₄ and R₅ is D;
R₆, R₇ and R₈ each independently represent H, D or F; and
R₉ and R₁₀ each independently represent H or F.

2. The compound according to claim 1, wherein at least two of R₁, R₂, R₃, R₄, R₅, R₆, R₇ and R₈ represent D.

3. The compound according to claim 1 or 2, wherein R₁, R₂ and R₃ are all H, or are all D.

4. The compound according to any one of claims 1 to 3, wherein R₄ and R₅ are both H, or are both D.

5. The compound according to any one of claims 1 to 4, wherein R₆, R₇ and R₈ are all H, or are all D, or are all F.

6. The compound according to claim 1, wherein the compound is selected from the group consisting of the compounds represented by any one of the following structural formulas:

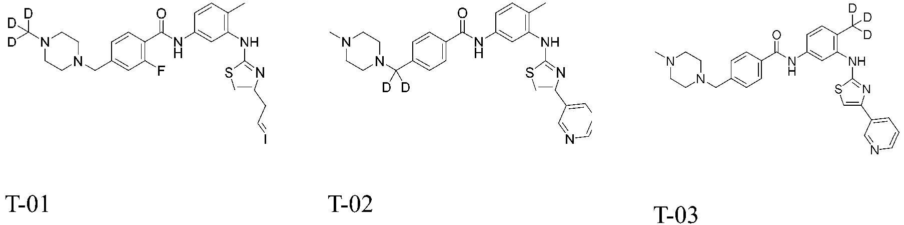

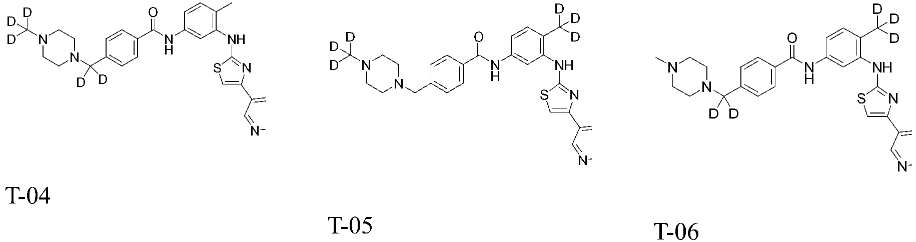

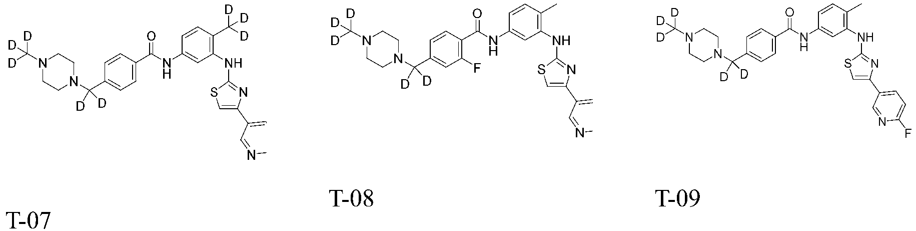

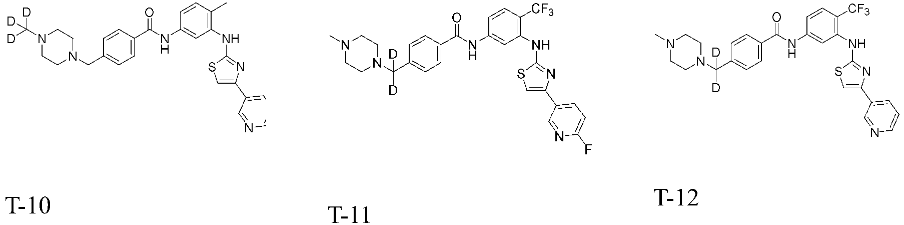

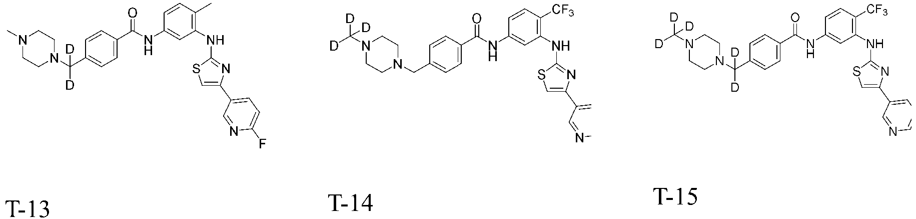

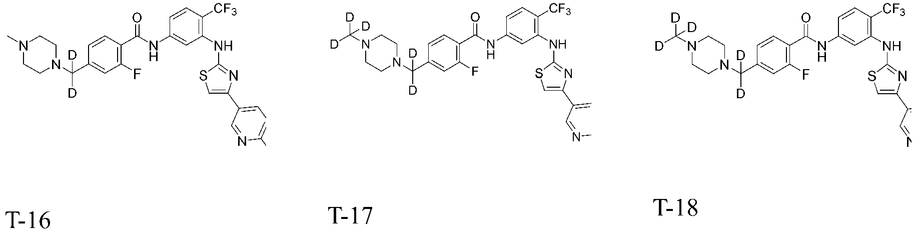

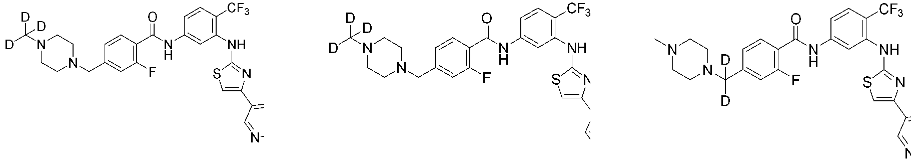

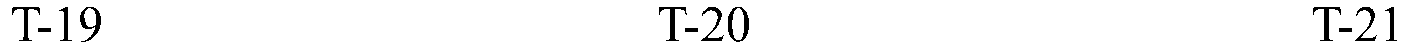

7. A pharmaceutical composition, comprising a prophylactically and/or therapeutically effective amount of the compound according to any one of claims 1 to 6, and a pharmaceutically acceptable carrier.

8. Use of the compound according to any one of claims 1 to 6 or the composition according to claim 7 in the manufacture of a medicament, wherein the medicament is used for treating a disease selected from the group consisting of neurodegenerative disease, cancer, progressive supranuclear palsy, mast cell activation syndrome, rheumatoid arthritis, asthma, Crohn's disease, sickle cell disease, and coronavirus disease 2019.

9. The use according to claim 8, wherein the neurodegenerative disease is selected from the group consisting of Alzheimer's disease, Parkinson's disease, progressive multiple sclerosis, and amyotrophic lateral sclerosis.

10. The use according to claim 8, wherein the cancer is selected from the group consisting of prostate cancer, pancreatic cancer, gastric cancer, liver cancer, multiple myeloma, and colorectal cancer.
